# EUROPÄISCHE PATENTSCHRIFT

(11) **EP 4 370 942 B1**
(45) Veröffentlichungstag und Bekanntmachung des Hinweises auf die Patenterteilung: **30.07.2025**
(21) Anmeldenummer: 22768677.1
(22) Anmeldetag: 22.08.2022
(51) Int. Cl.: G01R 33/34, G01R 33/3415

(54) **DENTALSPULE UND MAGNETRESONANZVORRICHTUNG FÜR DIE BILDGEBUNG EINER KIEFERREGION**
DENTAL COIL AND MAGNETIC RESONANCE DEVICE FOR IMAGING A JAW REGION
BOBINE DENTAIRE ET DISPOSITIF À RÉSONANCE MAGNÉTIQUE POUR L'IMAGERIE D'UNE RÉGION DE MÂCHOIRE

(30) Priorität: 17.09.2021 DE 102021210304
(43) Veröffentlichungstag der Anmeldung: 22.05.2024
(73) Patentinhaber: Siemens Healthineers AG, 91301 Forchheim (DE); Dentsply Sirona Inc., York, PA 17401 (US); Sirona Dental Systems GmbH, 64625 Bensheim (DE)
(72) Erfinder: MÜLLER, Edgar, 91336 Heroldsbach (DE); BIBER, Stephan, 91056 Erlangen (DE); DENNERT, Sebastian, 91475 Lonnerstadt (DE); GEBHARDT, Matthias, 91052 Erlangen (DE); GEISSNER, Marco, 97230 Estenfeld (DE); GREISER, Andreas, 91054 Erlangen (DE); LANZ, Titus, 97084 Würzburg (DE); LAUER, Lars, 91077 Neunkirchen (DE); ODOJ, Florian, 97209 Veitshöchheim (DE); ROTHARD, Jörg, 96123 Litzendorf (DE); ULRICI, Johannes, 64285 Darmstadt (DE); WICHMANN, Tobias, 97222 Rimpar (DE); WUNDRAK, Stefan, 64625 Bensheim (DE); ZELLER, Mario, 91054 Erlangen (DE)
(74) Vertreter: Siemens Healthineers Patent Attorneys
(86) Internationale Anmeldenummer: PCT/EP2022/073273
(87) Internationale Veröffentlichungsnummer: WO 2023/041289

(56) Entgegenhaltungen:
- EP-A1- 3 489 704
- JP-A- H0 329 639
- US-A1- 2012 288 820
- ANONYM: "Mandibula 15-Kanal Dental Spule - Noras MRI Products", 2017, Online, pages 1 - 74, XP055980776, Retrieved from the Internet <URL:https://docplayer.org/132670679-Mandibula-15-kanal-dental-spule-1-5t-si-t-si.html> [retrieved on 20221114]
- GRADL J ET AL: "Application of a Dedicated Surface Coil in Dental MRI Provides Superior Image Quality in Comparison with a Standard Coil", CLINICAL NEURORADIOLOGY, SPRINGER BERLIN HEIDELBERG, BERLIN/HEIDELBERG, vol. 27, no. 3, 11 February 2016 (2016-02-11), pages 371 - 378, XP036308711, ISSN: 1869-1439, [retrieved on 20160211], DOI: 10.1007/S00062-016-0500-9
- SEDLACIK JAN ET AL: "Optimized 14+1 receive coil array and position system for 3D high-resolution MRI of dental and maxillomandibular structures", DENTO-MAXILLO-FACIAL RADIOLOGY, vol. 45, no. 1, 2016, SE, pages 20150177, XP055981414, ISSN: 0250-832X, DOI: 10.1259/dmfr.20150177

## Beschreibung

Die Erfindung betrifft eine Dentalspule, welche dazu ausgebildet ist, hochfrequente Signale in einem Frequenz- und Leistungsbereich einer Magnetresonanzmessung einer Kieferregion eines Patienten zu empfangen. Weiterhin betrifft die Erfindung eine Magnetresonanzvorrichtung mit einer Dentalspule, wobei die Magnetresonanzvorrichtung dazu ausgebildet ist, eine Bildgebung einer Kieferregion eines Patienten durchzuführen.

Erkrankungen der Zähne und des Zahnhalteapparats, wie z. B. Karies oder Parodontitis, werden heutzutage üblicherweise mit röntgenbasierten Bildgebungsverfahren diagnostiziert. Dabei kommen vor allem konventionelle oder digitale Röntgen-Projektionsverfahren, sowie kürzlich auch dreidimensionale Röntgenverfahren, zum Einsatz. Ein Beispiel für ein dreidimensionales Röntgenverfahren stellt die digitale Volumentomographie dar, welche für eine Bildgebung von Zähnen und des Viscerocraniums eingesetzt werden kann.

Ein großer Nachteil von Röntgenverfahren ist die Notwendigkeit des Einsatzes von ionisierender Strahlung für die Bildgebung. Ein Bildgebungsverfahren, welches ionisierende Strahlen vermeidet, stellt die Magnetresonanztomografie dar. Diese ermöglicht typischerweise einen besseren Weichgewebekontrast als Röntgenverfahren und unterstützt standardmäßig eine dreidimensionale Bildgebung eines Untersuchungsobjekts. Weiterhin ermöglicht die Magnetresonanztomografie eine Bildgebung von Zysten sowie eine Erkennung einer Degradation von Dentin, bevor dies durch ein Röntgenverfahren erkennbar wird. Die Magnetresonanztomografie stellt somit eine potenzielle Alternative zu bekannten Röntgenverfahren bei der Bildgebung einer Gebissregion und/oder einer Kieferregion sowie der Diagnose von Zahnerkrankungen des Untersuchungsobjekts dar.

Die Magnetresonanztomografie ist ein bekanntes Bildgebungsverfahren, mit welchem Magnetresonanzbilder eines Inneren des Untersuchungsobjekts erzeugt werden können. Bei der Durchführung einer Magnetresonanzbildgebung wird das Untersuchungsobjekt üblicherweise in einem starken, statischen und homogenen Grundmagnetfeld (B0-Magnetfeld) einer Magnetresonanzvorrichtung positioniert. Das Grundmagnetfeld kann magnetische Feldstärken von 0,2 Tesla bis 7 Tesla aufweisen, sodass sich Kernspins des Untersuchungsobjekts entlang des Grundmagnetfeldes ausrichten. Um sogenannte Kernspinresonanzen auszulösen, werden hochfrequente Signale, sogenannte Anregungsimpulse (B1-Magnetfeld), in das Untersuchungsobjekt eingestrahlt. Jeder Anregungsimpuls bewirkt eine Abweichung einer Magnetisierung bestimmter Kernspins des Untersuchungsobjekts von dem Grundmagnetfeld um einen Betrag, welcher auch als Flipwinkel bekannt ist. Ein Anregungsimpuls kann dabei ein magnetisches Wechselfeld mit einer Frequenz aufweisen, welche der Larmorfrequenz bei der jeweiligen statischen Magnetfeldstärke entspricht. Die angeregten Kernspins können eine rotierende und abklingende Magnetisierung (Kernspinresonanz) aufweisen, welche sich mittels spezieller Antennen als Magnetresonanzsignal erfassen lässt. Zur räumlichen Kodierung der Kernspinresonanzen des Untersuchungsobjekts können dem Grundmagnetfeld magnetische Gradientenfelder überlagert werden.

Die empfangenen Magnetresonanzsignale werden typischerweise digitalisiert und als komplexe Werte in einer k-Raum-Matrix gespeichert. Diese k-Raum-Matrix kann als Grundlage für eine Rekonstruktion von Magnetresonanzbildern sowie eine Bestimmung von Spektroskopiedaten verwendet werden. Die Rekonstruktion eines Magnetresonanzbilds erfolgt typischerweise mittels einer mehrdimensionalen Fourier-Transformation der k-Raum-Matrix.

Die Magnetresonanztomografie eignet sich aufgrund der Vermeidung von ionisierender Strahlung insbesondere für eine kontinuierliche diagnostische Überwachung von Zahnerkrankungen und/oder einer Zahnentwicklung im Rahmen einer longitudinalen Bildgebungsstudie. Bei longitudinalen Bildgebungsstudien wird üblicherweise eine Mehrzahl von Bildgebungsuntersuchungen durchgeführt, um eine Progression einer Erkrankung oder einen Erfolg einer therapeutischen Behandlung über einen vorbestimmten Zeitraum zu bestimmen. Diagnostisch relevante Bereiche der Kieferregion eines Patienten, wie z. B. eine Mundhöhle, ein Gebiss, ein Zahnbogen oder ein Zahn, stellen jedoch ein geringes Volumen bereit, welches für eine Erzeugung von Magnetresonanzsignalen zur Verfügung steht. Ferner weisen konventionelle Volumen- und Oberflächenspulen, wie z. B. Kopfspulen und Auflegespulen, einen relativ großen Abstand zu dem diagnostisch relevanten Bereich auf. Ein großer Abstand kann jedoch ein Signal-zu-Rausch Verhältnis von erfassten Magnetresonanzsignalen erhöhen und somit eine Qualität von daraus rekonstruierten Magnetresonanzbildern des Gebisses des Patienten reduzieren.

Die Schrift "Mandibula 15-Kanal Dental Spule - Noras MRI Products", Internet, 2017, Seiten 1-74, XP055980776, URL: https://docplayer.org/132670679-Mandibula-15-kanal-dental-spule-1-5t-si-t-si.html, beschreibt eine 15-Kanal Dental Spule zur Bildgebung von Kiefer, Zähnen und Pulpa.

Die Druckschrift EP 3 489 704 A1 betrifft eine lokale MRT-Spule mit mindestens zwei Spulenelementen für eine dentale MRT-Messung eines Patientenkopfes.

In der Druckschrift US 2012/288820 A1 wird ein MRI-System mit einer Radiofrequenz-Spuleneinheit beschrieben. Die Radiofrequenz-Spuleneinheit kann berührend oder berührungsfrei an einem Abschnitt eines Patienten angebracht werden und ermöglicht einen Empfang eines MR-Signals des Abschnitts des Patienten.

Die Druckschrift JP H03 29639 A offenbart eine Lokalspule, welche integral aus einer leitenden Spule und einem Formgedächtnismaterial hergestellt ist und eine freie Verbiegung für die Anpassung an eine äußere Form einer Testperson erlaubt.

Es ist daher eine Aufgabe der Erfindung, eine Dentalspule bereitzustellen, welche eine Aufnahme von Magnetresonanzbildern der Kieferregion des Patienten mit hoher Qualität ermöglicht.

Diese Aufgabe wird durch den Gegenstand des unabhängigen Patentanspruchs erfindungsgemäß gelöst. Vorteilhafte Ausführungsformen und zweckmäßige Weiterbildungen sind Gegenstand der Unteransprüche.

Die erfindungsgemäße Dentalspule umfasst ein erstes Element, wobei das erste Element aus einem formsteifen Material besteht und eine Aussparung umfasst, welche dazu geeignet ist, eine Mundregion und/oder eine Nasenregion eines Patienten bei anwendungsgemäßer Positionierung der Dentalspule an einer Kieferregion des Patienten aufzunehmen.

Ein formsteifes Material kann ein beliebiges Material darstellen, welches bei einer anwendungsgemäßen Positionierung der Dentalspule an einer Kieferregion des Patienten eine vernachlässigbare Verformung aufweist. Vorzugsweise weist das formsteife Material eine möglichst geringe Wechselwirkung mit magnetischen Feldern auf, sodass das erste Element im Wesentlichen undetektierbar für eine Magnetresonanzmessung ist. Beispiele für geeignete, formsteife Materialien stellen Kunststoffe wie Silikone, Polyester und Polycarbonate, aber auch verschiedene Keramiken, wie Aluminiumoxid oder Zirconiumoxid, dar. Darüber hinaus kommen selbstverständlich auch andere Materialien, wie z. B. Glas oder glasfaserverstärkte Kunststoffe, in Frage. Die Aussparung des ersten Elements kann ein Loch oder einen Durchbruch darstellen, welcher ein Atmen des Patienten und/oder eine Belüftung der Mund- und/oder Nasenregion bei anwendungsgemäßer Positionierung der Dentalspule an der Kieferregion ermöglicht. Das erste Elemente kann insbesondere als ein Rahmen verstanden werden, welcher die Mund- und/oder Nasenregion des Patienten umschließt und/oder eine Positionierung der Dentalspule an der Kieferregion des Patienten vereinfacht.

Die erfindungsgemäße Dentalspule umfasst ferner ein zweites Element, wobei das zweite Elemente ein flexibles Element aufweist, welches dazu ausgebildet ist, ein Nachformen der Kieferregion des Patienten mittels des flexiblen Elements zu ermöglichen.

Ein flexibles Element ist vorzugsweise reversibel verformbar. Das zweite Element kann einstückig aus dem flexiblen Element gefertigt sein oder Segmente aufweisen, welche eine reversible Verformung des flexiblen Elements ermöglichen. Ein Segment des flexiblen Elements kann z. B. ein Scharnier, ein Gelenk, ein Lager oder dergleichen darstellen. Es ist vorstellbar, dass das flexible Element mittels eines Segments reversibel verformbar ist. Das Segment kann dabei ein elastisches und/oder ein formsteifes Material aufweisen.

Vorzugsweise weist das flexible Element ein plastisch oder elastisch vorformbares Material auf. Das flexible Element kann auch gänzlich aus einem plastisch oder elastisch verformbaren Material gefertigt sein. Beispiele für solche Materialien sind Kunststoffe wie Polyethen, Polyurethan, Polyamid und Polyester. Daneben sind auch Materialien auf natürlicher Basis, wie z. B. Kautschuk oder Fasermaterialien, vorstellbar. In einer bevorzugten Ausführungsform ist das flexible Element als Schaumstoff, Faserstoff oder dergleichen ausgeführt, um eine geringe Dichte und somit ein geringes Gewicht zu erreichen. Das flexible Element kann derart verformbar sein, dass das zweite Element individuellen Geometrien einer Mehrzahl von unterschiedlichen Kieferregionen verschiedener Patienten nachgeformt werden kann. In einer bevorzugten Ausführungsform ist das flexible Element mittels einer manuellen Krafteinwirkung eines Bedieners einer Magnetresonanzvorrichtung oder des Patienten verformbar. Ein Nachformen der Kieferregion des Patienten mittels des flexiblen Elements kann insbesondere ein Verformen und/oder ein Anpassen einer Oberflächenkontur des zweiten Elements umfassen. Das zweite Element kann dabei in Kontakt mit einer Hautoberfläche der Kieferregion gebracht werden (z. B. durch Andrücken). Es ist aber ebenso vorstellbar, dass das zweite Element der Kieferregion des Patienten ohne Kontakt mit der Hautoberfläche nachgeformt wird.

Das zweite Element kann in einer anwendungsgemäßen Position der Dentalspule an der Kieferregion des Patienten im Wesentlichen tangential zu einer Wange des Patienten, insbesondere parallel zu einer Reihe von benachbarten Backenzähnen des Patienten, ausgerichtet sein. Das zweite Element kann ferner eine Krümmung aufweisen, welche einer Krümmung der Wange und/oder eines Teils des Zahnbogens des Patienten nachgeformt ist.

Die erfindungsgemäße Dentalspule ist insbesondere dazu ausgebildet, das erste Element und das zweite Element zeitgleich an der Kieferregion des Patienten zu positionieren und/oder zu halten. Dadurch lassen sich mittels des ersten Elements und des zweiten Elements Magnetresonanzsignale der Kieferregion im Rahmen einer Magnetresonanzmessung der Kieferregion des Patienten erfassen.

Das erste Elemente und das zweite Element der erfindungsgemä-ßen Dentalspule weisen eine Antenne auf, welche dazu ausgebildet ist, hochfrequente Signale in einem Frequenz- und Leistungsbereich einer Magnetresonanzmessung zu empfangen.

Eine Antenne kann ein Koppelelement zwischen in Signalleitern geführten und ungeführten, d. h. in einem Freiraum befindlichen, elektromagnetischen Wellen darstellen. Die Antenne ist insbesondere dazu ausgebildet, elektromagnetische Wellen im Bereich einer Magnetresonanzfrequenz eines magnetresonanzaktiven Atomkerns zu empfangen. Für Magnetresonanzmessungen relevante elektromagnetische Wellen können hochfrequente Signale bzw. Magnetresonanzsignale sein, welche Frequenzen zwischen 1 und 500 MHz, vorzugsweise zwischen 10 und 300 MHz, umfassen. Die Magnetresonanzsignale üblicher zu untersuchender Atomkerne können eine geringe Leistung von einigen Mikrowatt bis mehrere Milliwatt aufweisen.

Ein Signalleiter ist vorzugsweise ein elektrisch leitender Draht. Der Draht des Signalleiters kann einen ovalen oder polygonalen Querschnitt aufweisen und dazu geeignet sein, die oben angegebenen Leistungen kontinuierlich zu übertragen. Es ist ebenso vorstellbar, dass der Signalleiter als eine Leiterbahn auf einer Leiterplatte ausgeführt ist und einen annähernd rechteckigen Querschnitt aufweist. Der Signalleiter kann aus Kupfer bestehen. Es sind aber auch andere elektrisch leitende Metalle, wie z. B. Gold oder Aluminium, vorstellbar.

Die Antenne der erfindungsgemäßen Dentalspule weist vorzugsweise einen Berührschutz auf, welcher ein Untersuchungsobjekt vor Spannungen und/oder Verbrennungen schützt. Der Signalleiter der Antenne kann hierfür z. B. eine Beschichtung und/oder eine Verkleidung aus Kunststoff aufweisen. Geeignete Kunststoffe stellen z. B. Polytetrafluorethylen (PTFE) oder diverse Polysiloxane dar. Bei dem Empfangen eines Magnetresonanzsignals kann in dem Signalleiter ein Strom induziert werden, welcher mittels einer geeigneten elektrischen Anschlussleitung an eine Magnetresonanzvorrichtung übertragen werden kann. Eine solche elektrische Anschlussleitung kann z. B. ein Koaxialkabel sein, welches eine Abschirmung aufweist, um elektromagnetische Einstreuung aus einer Umgebung zu vermeiden.

Es ist vorstellbar, dass die Dentalspule eine elektronische Schaltung aufweist, welche mit der Antenne verbunden ist. Die elektronische Schaltung kann ein elektronisches Bauelement oder einen Zusammenschluss von mehreren elektronischen Bauelementen, wie z. B. Transistoren, Widerständen, Kondensatoren, Dioden, Leiterbahnen und dergleichen, umfassen. Die elektronische Schaltung kann insbesondere eine Schutzschaltung aufweisen, welche dazu geeignet ist, die Antenne gegen eine Überlastung zu schützen. Für die Vermeidung magnetischer Anziehungskräfte, stehender Wellen, Aufheizung und vergleichbarer, unerwünschter Effekte, kann die elektronische Schaltung einen hohen Anteil nicht-magnetischer Materialien sowie entsprechende Mantelwellensperren und/oder Baluns aufweisen. Die elektronische Schaltung weist vorzugsweise eine Leiterplatte (PCB) oder ein vergleichbares Substrat auf, welches dazu geeignet ist, die elektronischen Bauelemente in einer vorbestimmten Position zueinander aufzunehmen.

Selbstverständlich kann die erfindungsgemäße Dentalspule mehrere Antennen gemäß einer oben beschriebenen Ausführungsform umfassen. Die Antennen können dabei voneinander beabstandet, aneinander angrenzend oder teilweise überlappend angeordnet sein. Die Antennen können ferner in Form eines Gitters oder einer Matrix angeordnet sein. Beispielsweise kann das erste Element eine erste Antenne oder eine erste Anzahl von Antennen aufweisen, während das zweite Element eine zweite Antenne oder eine zweite Anzahl von Antennen aufweist.

Es ist weiterhin vorstellbar, dass die erfindungsgemäße Dentalspule zumindest eine Antenne aufweist, welche dazu ausgelegt ist, ein hochfrequentes Signal in eine Richtung des Untersuchungsobjekts, wie z. B. eine Kieferregion des Patienten, auszustrahlen. Das von der zumindest einen Antenne ausgestrahlte, hochfrequente Signal kann in Abhängigkeit des Grundmagnetfelds einer Magnetresonanzvorrichtung beispielsweise in einem Leistungsbereich von wenigen Watt bis mehreren Kilowatt liegen. Das von der zumindest einen Antenne ausgestrahlte, hochfrequente Signal kann insbesondere ein B1-Magnetfeld darstellen. Ein Teil der Dentalspule mit der zumindest einen Antenne kann beispielsweis eine Sendeeinheit der Dentalspule darstellen.

In einer bevorzugten Ausführungsform weist das erste Element eine Anzahl von Antennen auf, welche dazu ausgebildet sind, Magnetresonanzsignale aus einer Kieferregion des Patienten zu empfangen. Die Anzahl von Antennen kann in das erste Elemente integriert und/oder eingebettet sein oder von diesem getragen werden. Das formsteife Material des ersten Elements kann dazu ausgebildet sein, einen Berührschutz für den Patienten gemäß einer oben beschriebenen Ausführungsform bereitzustellen.

Eine Aussparung kann einen ovalen oder einen polygonalen Ausschnitt in dem ersten Element darstellen. Die Aussparung ist vorzugsweise durch einen Abschnitt des ersten Elements charakterisiert, welcher frei von Signalleitern ist. Die Aussparung kann insbesondere dazu ausgelegt sein, einen Freiraum für eine Nase und/oder einen Mund des Patienten bereitzustellen, wenn die Dentalspule in einer anwendungsgemäßen Position für die Durchführung einer Magnetresonanzmessung an der Kieferregion des Patienten positioniert ist. Ein Kontakt zwischen der Nase und/oder des Mundes des Patienten und dem ersten Element kann somit vermieden werden. In einer bevorzugten Ausführungsform sind die Antenne oder die Antennen des ersten Elements derart angeordnet, dass die Aussparung frei von Signalleitern ist. Hierfür können die Antenne oder die Antennen die Aussparung beispielsweise schlaufenförmig umgeben. Das erste Element kann eine oder mehrere Aussparungen aufweisen. Beispielsweise kann das erste Element eine erste Aussparung für die Nase und eine zweite Aussparung für den Mund des Patienten aufweisen. Es ist aber ebenso vorstellbar, dass das erste Element eine einzige Aussparung aufweist, welche sowohl die Nase als auch den Mund des Patienten aufnehmen kann.

Das erste Element kann bei anwendungsgemäßer Positionierung der Dentalspule an der Kieferregion des Patienten im Wesentlichen parallel zu einer Frontalebene des Patienten ausgerichtet sein. Es ist ebenso vorstellbar, dass das erste Element bei anwendungsgemäßer Positionierung der Dentalspule im Wesentlichen parallel zu einem Zahnbogen des Patienten, insbesondere parallel zu einer Reihe von Schneidezähnen Patienten, ausgerichtet ist. Das erste Element kann bei anwendungsgemäßer Positionierung der Dentalspule an einer Hautoberfläche der Kieferregion des Patienten anliegen oder einen vorbestimmten Abstand von weniger als zwei Zentimetern, weniger als einem Zentimeter oder weniger als fünf Millimetern zu der Hautoberfläche der Kieferregion des Patienten aufweisen.

Vorzugsweise weist das zweite Element eine Antenne oder eine Anzahl von Antennen auf, welche von dem zweiten Element gehalten werden. Die Antenne oder die Anzahl von Antennen können beispielsweise in das zweite Element integriert und/oder eingebettet sein und/oder mit diesem verbunden sein. Vorzugsweise sind die Antenne oder die Anzahl von Antennen reversibel verformbar, um der Kieferregion des Patienten nachgeformt zu werden. Das zweite Element als ein Flügel oder eine Flanke ausgestaltet sein, welche eine linke Wange und/oder eine rechte Wange des Patienten bei anwendungsgemäßer Positionierung der Dentalspule an der Kieferregion zumindest teilweise umschließen. Das zweite Element kann insbesondere eine erste Flanke und eine zweite Flanke aufweisen, wobei die erste Flanke der linken Wange des Patienten nachgeformt ist und wobei die zweite Flanke der rechten Wange des Patienten nachgeformt ist. Es ist jedoch ebenso vorstellbar, dass das zweite Element lediglich eine Flanke aufweist. Die Dentalspule kann in diesem Fall auch ein drittes Element aufweisen, welches bei anwendungsgemäßer Positionierung der Dentalspule an der dem zweiten Element gegenüberliegenden Wange des Patienten angeordnet und dieser nachgeformt ist. Das dritte Element kann analog zu einer Ausführungsform des zweiten Elements ausgestaltet sein.

In einer bevorzugten Ausführungsform weist die erfindungsgemäße Dentalspule eine Halterung auf, welche dazu ausgebildet ist, das erste Element und/oder das zweite Element in einer anwendungsgemäßen Position an der Kieferregion des Patienten zu halten und/oder zu positionieren. In einem einfachen Beispiel kann die Halterung zwei Streben oder Trägerelemente aufweisen, welche bei anwendungsgemäßer Positionierung der Dentalspule den Kopf des Patienten auf einer linken Seite und einer rechten Seite des Kopfs flankieren oder einschließen. Die Halterung kann ferner ein Halteelement aufweisen, welches dazu ausgebildet ist, eine Feinjustierung einer Orientierung und/oder einer Position des ersten Elements und/oder des zweiten Elements relativ zu der Kieferregion des Patienten vorzunehmen. Es ist jedoch ebenso vorstellbar, dass die Halterung eine Positionierungseinheit umfasst, welche dazu ausgebildet ist, eine grobe Positionierung der Dentalspule relativ zu dem Patienten und/oder einer Patientenlagerungsvorrichtung vorzunehmen. Eine Patientenlagerungsvorrichtung kann dabei eine beliebige Struktur darstellen, welche den Patienten bei einer Einhaltung einer vorbestimmten Position und/oder Körperhaltung während einer Magnetresonanzmessung unterstützt. Die Patientenlagerungsvorrichtung kann insbesondere dazu ausgebildet sein, eine Körperregion des Patienten in einer sitzenden, einer liegenden und/oder einer stehenden Körperhaltung zu stützen.

Durch ein Bereitstellen des ersten Elements mit einer Aussparung wird eine Beeinträchtigung einer Atmung des Patienten während einer Magnetresonanzmessung der Kieferregion des Patienten auf vorteilhafte Weise reduziert oder vermieden. Ferner lässt sich eine Auflagefläche der Dentalspule auf der Hautoberfläche der Kieferregion des Patienten auf eine Fläche des ersten Elements reduzieren, was zu einem erhöhten Komfort des Patienten beiträgt und ein Risiko eines Abbruchs der Magnetresonanzmessung durch den Patienten reduziert. Weiterhin lässt sich eine manuelle Positionierung der Dentalspule an der Kieferregion des Patienten vereinfachen, da eine Orientierung sowie eine Relativposition der Dentalspule zu der Kieferregion des Patienten bereits durch die Aussparung in dem ersten Element vorgegeben sind. Dadurch kann auf vorteilhafte Weise eine Zeitersparnis bei der Positionierung der Dentalspule erreicht werden.

Durch ein Bereitstellen des zweiten Elements, welches der Kieferregion des Patienten nachgeformt werden kann, lassen sich eine oder mehrere Antennen der Dentalspulen auf vorteilhafte Weise in einem besonders geringen Abstand und/oder individuell angepasst an der Kieferregion positionieren. Somit lässt sich ein Signal-zu-Rausch Verhältnis empfangener Magnetresonanzsignale auf vorteilhafte Weise erhöhen.

In einer Ausführungsform der erfindungsgemäßen Dentalspule ist das zweite Element unabhängig von dem ersten Element positionierbar.

Die erfindungsgemäße Dentalspule weist eine Halterung auf, welche dazu ausgebildet sind, das erste Element und/oder das zweite Element in der anwendungsgemäßen Position an der Kieferregion des Patienten zu halten. Das erste Element und das zweite Element können z. B. mit verschiedenen Abschnitten der Halterung und/oder des Halteelements mechanisch verbunden sein, welche unabhängig voneinander reversibel verformbar sind. Es ist ebenso vorstellbar, dass die verschiedenen Abschnitte der Halterung und/oder des Halteelements mittels eines Anpassungsmechanismus, wie z. B. eines Scharniers, eines Gelenks oder eines vergleichbaren Mechanismus, relativ zueinander positionierbar und/oder orientierbar sind.

Eine unabhängige Positionierbarkeit des ersten Elements und des zweiten Elements kann bedeuten, dass das erste Element und das zweite Element getrennt voneinander, insbesondere ohne eine mechanische Verbindung zueinander, vorliegen. In diesem Fall kann eine Position des zweiten Elements unabhängig von einer Position des ersten Elements einstellbar sein. Es kann aber auch bedeuten, dass das zweite Element mechanisch mit dem ersten Element verbunden ist. In diesem Fall kann insbesondere eine Form und/oder eine Relativposition zumindest eines Teils des zweiten Elements mittels des flexiblen Elements gegenüber dem ersten Element anpassbar sein.

Eine unabhängige Positionierung des ersten Elements und des zweiten Elements kann eine Anpassung der Dentalspule an eine individuelle Geometrie einer Kieferregion vereinfachen und/oder einen Abstand zwischen der Kieferregion und der Dentalspule verringern. Dadurch lässt sich ein Signal-zu-Rausch Verhältnis der Dentalspule auf vorteilhafte Weise erhöhen.

In einer alternativen Ausführungsform der erfindungsgemäßen Dentalspule sind das erste Element und das zweite Element mechanisch miteinander verbunden, wobei das zweite Element von dem ersten Element gehalten wird.

Es ist vorstellbar, dass das erste Element und das zweite Element mittels eines Scharniers oder eines Gelenks miteinander verbunden sind, um eine Anpassung und/oder eine Anformung der Dentalspule an die Kieferregion des Patienten zu verbessern. Es ist aber ebenso vorstellbar, dass die Möglichkeit einer Anpassung und/oder einer Anformung der Dentalspule an die Kieferregion des Patienten bereits mittels des flexiblen Elements gegeben ist. Eine mechanische Verbindung zwischen dem ersten Element und dem zweiten Element kann durch eine beliebige stoffschlüssige, kraftschlüssige und/oder formschlüssige Verbindung realisiert sein. Selbstverständlich kann auch ein drittes Element mechanisch mit dem ersten Element verbunden oder unabhängig von dem ersten Element positionierbar sein.

Durch eine mechanische Verbindung des ersten Elements mit dem zweiten Element lässt sich die Dentalspule besonders einfach an der Kieferregion des Patienten positionieren. Dadurch kann ein Zeitaufwand für eine Vorbereitung der Magnetresonanzmessung der Kieferregion auf vorteilhafte Weise reduziert werden.

In einer weiteren Ausführungsform weist die erfindungsgemäße Dentalspule ferner einen Anpassungsmechanismus auf, welcher dazu ausgebildet ist, ein Anpassen einer Form des flexiblen Elements vorzunehmen, um das zweite Element der Kieferregion des Patienten nachzuformen.

In einer Ausführungsform lässt sich das zweite Element mittels des Anpassungsmechanismus an die äußere Gestalt der Kieferregion des Patienten anformen. Der Anpassungsmechanismus kann hierfür einen Führungsmechanismus, einen Spannmechanismus, einen Klemmmechanismus, einen Biegemechanismus, einen Zugmechanismus oder dergleichen aufweisen, welcher dazu ausgelegt ist, das zweite Element in der anwendungsgemäßen Position an der Kieferregion des Patienten zu verformen und/oder einen Biegeradius des zweiten Elements in Abhängigkeit einer Kontur der Kieferregion des Patienten einzustellen. Es ist vorstellbar, dass der Anpassungsmechanismus mechanisch mit der Halterung der Dentalspule verbunden ist.

Vorzugsweise weist der Anpassungsmechanismus ein Stellelement auf, welches dazu ausgebildet ist, eine Kraft auf das zweite Element zu übertragen. Ein Stellelement kann beispielsweise eine Schraube, ein Stift, ein Bolzen und/oder ein elastisches Element darstellen, welches zwischen der Halterung und dem zweiten Element mittels des Anpassungsmechanismus positionierbar gelagert und/oder einspannbar ist. Dabei kann eine auf den Anpassungsmechanismus ausgeübte Kraft mittels des Stellelements auf das zweite Element übertragen werden, sodass das zweite Element oder ein Abschnitt des zweiten Elements relativ zu der Kieferregion des Patienten positioniert wird. Der Anpassungsmechanismus kann insbesondere eine Mehrzahl von Stellelementen aufweisen, welche dazu ausgebildet sind, eine Oberflächenkontur einer der Kieferregion zugewandten Seite des zweiten Elements an eine Oberflächenkontur der Kieferregion des Patienten anzupassen. Das zweite Element kann dabei in Kontakt mit der Hautoberfläche der Kieferregion des Patienten treten. Es ist aber ebenso vorstellbar, dass ein vorbestimmter Abstand von weniger als zwei Zentimetern, weniger als einem Zentimeter oder weniger als 5 Millimetern zwischen dem zweiten Element und der Hautoberfläche der Kieferregion des Patienten aufrechterhalten wird.

Das Stellelement kann ferner ein elastisches Element, wie z. B. ein synthetisches oder natürliches Elastomer, eine Feder, einen Schaumstoff oder dergleichen, aufweisen. Das elastische Element kann bei anwendungsgemäßer Positionierung der Dentalspule an der Kieferregion zwischen dem zweiten Element und der Positionierungseinheit und/oder der Halterung eingespannt sein und eine elastische Rückstellkraft auf das zweite Element übertragen. Vorzugsweise wird das zweite Element mittels der elastischen Rückstellkräfte des elastischen Elements in Richtung der Kieferregion des Patienten ausgelenkt und dabei der Kieferregion des Patienten nachgeformt. Das elastische Element kann aber auch ein Halteelement darstellen, welches das zumindest das zweite Element, aber auch das erste Element und/oder das dritte Element, in einer anwendungsgemä-ßen Position an der Kieferregion des Patienten hält.

Ein Vorteil des erfindungsgemäßen Anpassungsmechanismus stellt eine besonders einfache und/oder zeiteffiziente Verringerung des Abstands zwischen einer oder mehrerer Antennen der Dentalspule zu der Kieferregion des Patienten dar. Dadurch lässt sich auf vorteilhafte Weise ein erhöhtes Signal-zu-Rausch Verhältnis empfangener Magnetresonanzsignale und/oder eine reduzierte Vorbereitungszeit einer Magnetresonanzmessung der Kieferregion der Patienten erreichen.

Gemäß einer weiteren Ausführungsform der erfindungsgemäßen Dentalspule weist das flexible Element ein intelligentes Material auf, welches dazu ausgebildet ist, eine Materialeigenschaft in Abhängigkeit eines vorbestimmten Anregungssignals zu verändern, wobei der Anpassungsmechanismus dazu ausgebildet ist, das Anpassen der Form des zweiten Elements mittels einer Übertragung des Anregungssignals auf das flexible Element zu unterstützen.

Ein intelligentes Material kann ein beliebiges Material darstellen, bei welchem eine Materialeigenschaft des zweiten Elements mittels eines geeigneten Anregungssignals in einer vorbestimmten Weise verändert wird. Eine Materialeigenschaft kann dabei insbesondere eine Verformbarkeit des intelligenten Materials betreffen. Weitere Beispiele für betroffene Materialeigenschaften des intelligenten Materials sind unter anderem eine Kohäsionskraft, eine Elastizität, eine Steifigkeit, eine Duktilität oder eine Festigkeit. Vorzugsweise ist eine Materialeigenschaft des intelligenten Materials mittels des Anregungssignals derart einstellbar, dass das zweite Element gegenüber einem unangeregten Referenzzustand unter Aufwenden eines reduzierten Kraftaufwands verformbar ist. Es ist jedoch ebenso vorstellbar, dass das intelligente Material des zweiten Elements in dem unangeregten Referenzzustand unter manuellem Kraftaufwand flexibel verformbar ist, während das intelligente Material während und/oder nach Applikation des Anregungssignals (angeregter Zustand) eine reduzierte Flexibilität und/oder Verformbarkeit aufweist. Ferner kann das intelligente Material in dem unangeregten Referenzzustand auch eine erhöhte Steifigkeit aufweisen, welche während und/oder nach der Applikation des Anregungssignals reduziert ist.

Ein Anregungssignal kann ein beliebiges elektrisches, thermisches und/oder elektromagnetisches Signal umfassen. Das Anregungssignal kann ferner eine mechanische Vibration und/oder eine chemische Reaktion umfassen. In einem Beispiel weist das intelligente Material ein Formgedächtnispolymer und/oder eine Formgedächtnislegierung auf, welche bei Überschreiten einer vorbestimmten Temperatur und/oder Bestrahlen mit elektromagnetischer Strahlung einer vorbestimmten Wellenlänge (z. B. hochfrequente Signale einer Magnetresonanzvorrichtung, Licht eines vorbestimmten Spektrums, etc.) eine vorbestimmte Form annehmen. In einem weiteren Beispiel ist das intelligente Material dazu ausgebildet, bei einer geringen Temperaturerhöhung (thermisches Anregungssignal), wie z. B. bei einem Kontakt mit der Hautoberfläche der Kieferregion, zu Schmelzen und eine verbesserte Anformung des zweiten Elements an die Kieferregion zu ermöglichen. Es ist ferner vorstellbar, dass das intelligente Material aufgrund eines Piezoeffekts oder inversen Piezoeffekts bei Anlegen einer elektrischen Spannung und/oder eines elektrischen Stroms verformt werden (vgl. Piezoelektrika). Daneben kann das zweite Element auch ein elektrorheologisches und/oder magnetorheologisches Fluid, ein elektrostriktives und/oder magnetostriktives Material und/oder ein Funktionspolymer aufweisen. Weiterhin kommen auch mikro- oder nanoverkapselte Materialien und Kohlenstoffnanoröhren als intelligente Materialien in Frage.

Durch den Einsatz von intelligenten Materialien lässt sich ein Aufwand für das Nachformen bzw. Anformen des zweiten Elements an die Kieferregion des Patienten, insbesondere einer Mehrzahl verschiedener Patienten, auf vorteilhafte Weise reduzieren. Dadurch lässt sich die Antenne des zweiten Elements auch bei einem erhöhten Patientenaufkommen reproduzierbar und effizient in einem geringen Abstand zu der Kieferregion des Patienten positionieren.

Gemäß einer weiteren Ausführungsform weist die erfindungsgemäße Dentalspule ferner eine Halterung auf, welche mechanisch mit dem ersten Element und/oder dem zweiten Element verbunden ist.

Vorzugsweise ist die Halterung mechanisch mit dem ersten Element und dem zweiten Element verbunden. Die Halterung kann gemäß einer oben beschriebenen Ausführungsform ausgestaltet sein. Die Halterung weist beispielsweise zwei Trägerelemente auf, welche den Kopf des Patienten bei anwendungsgemäßer Positionierung der Dentalspule von zwei Seiten flankieren.

Das erste Element und/oder das zweite Element sind mittels der Halterung relativ zu dem Patienten positionierbar, wobei die Halterung dazu ausgebildet ist, das erste Element und/oder das zweite Element in der anwendungsgemäßen Position an der Kieferregion des Patienten zu halten.

Die Halterung kann gemäß einer oben beschriebenen Ausführungsform mittels der Positionierungseinheit relativ zu dem Patienten und/oder der Patientenlagerungsvorrichtung positioniert werden. In einer Ausführungsform weist die Halterung ein Halteelement auf, welches mechanisch mit einem oder mehreren Trägerelementen der Halterung sowie dem ersten Element und/oder dem zweiten Element verbunden ist. Es ist vorstellbar, dass die Halterung einen Mechanismus aufweist, welcher dazu ausgebildet ist, eine Position und/oder eine Orientierung des Halteelements bzw. des ersten Elements und/oder des zweiten Elements relativ zu dem Patienten und/oder der Patientenlagerungsvorrichtung einzustellen. Der Mechanismus kann entsprechend einer oben beschriebenen Ausführungsform z. B. als ein Gelenk, ein Scharnier, aber auch ein Drehlager, ein Wälzlager, ein Gleitlager oder dergleichen ausgeführt sein. Die Halterung stellt insbesondere eine erforderliche mechanische Stabilität bereit, um das erste Element und/oder das zweite Element anwendungsgemäß an der Kieferregion des Patienten zu positionieren und zu halten.

Mittels der erfindungsgemäßen Halterung lässt sich die Dentalspule auf robuste und/oder reproduzierbare Weise an der Kieferregion des Patienten positionieren. Dadurch kann eine Relativbewegung zwischen der Dentalspule und dem Patienten während einer Magnetresonanzmessung der Kieferregion auf vorteilhafte Weise vermieden werden. Dies kann insbesondere von Bedeutung sein, wenn das erste Element und/oder das zweite Element in einem geringen Abstand zu der Hautoberfläche der Kieferregion des Patienten positioniert sind, um eine Einschränkung eines Komforts des Patienten durch aufsetzen der Dentalspule auf der Hautoberfläche zu vermeiden.

Gemäß einer weiteren Ausführungsform weist die erfindungsgemäße Dentalspule eine Positionierungseinheit auf, welche dazu ausgebildet ist, eine Position und/oder Orientierung der Halterung relativ zu dem Patienten und/oder einer Patientenlagerungsvorrichtung einzustellen.

Die Positionierungseinheit ist vorzugsweise als ein Teil der Halterung ausgestaltet. Die Positionierungseinheit ist dazu ausgebildet, die Halterung mit der Dentalspule, insbesondere das erste Element und/oder das zweite Element, relativ zu der Kieferregion des Patienten zu positionieren. Die Positionierungseinheit kann einen beliebigen Mechanismus aufweisen, welcher dazu konfiguriert ist, eine Position und/oder eine Orientierung der Dentalspule, aber ebenso des ersten Elements und/oder des zweiten Elements, relativ zu dem Patienten anzupassen. Ein Anpassen einer Position und/oder einer Orientierung der Dentalspule kann z. B. einen Transport der Dentalspule entlang zumindest einer Raumrichtung und/oder eine Ausrichtung der Dentalspule entlang zumindest einer Drehachse umfassen. Vorzugsweise ist die Positionierungseinheit dazu ausgebildet, die Dentalspule entlang einer Mehrzahl von Raumrichtungen und/oder Drehachsen zu bewegen. Die Positionierungseinheit kann mechanisch mit zumindest einem Teil der Halterung verbunden sein.

In einer Ausführungsform ist die Positionierungseinheit dazu ausgebildet, die Dentalspule relativ zu einer Patientenlagerungsvorrichtung zu transportieren. Die Positionierungseinheit und/oder die Halterung können dabei zumindest eine erste Fläche aufweisen, welche in einem Abstand zu einer Patientenlagerungsfläche der Patientenlagerungsvorrichtung unbeweglich gelagert ist. Ein gleichförmiger Spalt zwischen der Patientenlagerungsvorrichtung und der ersten Fläche kann dabei einen Führungsmechanismus aufweisen oder als ein solcher ausgebildet sein. Der Führungsmechanismus kann beispielsweise, ein Rollensystem, ein Schienensystem, eine Linearführung, ein Teleskopsystem oder dergleichen aufweisen. Die Positionierungseinheit kann insbesondere dazu ausgebildet sein, die Dentalspule entlang einer Längsachse der Patientenlagerungsvorrichtung mittels des Führungsmechanismus zu positionieren. Es ist weiterhin vorstellbar, dass die Positionierungseinheit eine zweite Fläche und/oder eine Leiste bzw. eine Stange aufweist, welche in einem vorbestimmten Abstand zu der ersten Fläche zwischen der ersten Fläche und der Patientenlagerungsfläche der Patientenlagerungsvorrichtung positioniert ist. Ein Spalt zwischen der ersten Fläche und der zweiten Fläche und/oder der Leiste kann wiederum einen Führungsmechanismus aufweisen oder als ein solcher ausgebildet sein. Der Führungsmechanismus kann dazu ausgebildet sein, eine Bewegung der Halterung relativ zu der Patientenlagerungsvorrichtung auf zumindest eine vorbestimmte Raumrichtung und/oder Bewegungstrajektorie zu beschränken. Es ist jedoch ebenso vorstellbar, dass der Führungsmechanismus die Bewegung der Halterung relativ zu der Patientenlagerungsvorrichtung auf zumindest zwei oder drei vorbestimmte Raumrichtungen und/oder Bewegungstrajektorien beschränkt. Die erste Fläche kann dazu ausgebildet sein, den Patienten während einer Magnetresonanzmessung aufzunehmen und/oder bei einer Wahrung einer für die Magnetresonanzmessung erforderlichen Haltung zu unterstützen.

In einer Ausführungsform weist die Positionierungseinheit einen Schwenkmechanismus auf, welcher dazu ausgebildet ist, die Halterung gegenüber einer Längsachse des Patienten und/oder der Magnetresonanzvorrichtung zu schwenken oder zu verkippen. Beispielsweise weist die erste Fläche ein Scharnier oder ein Gelenk auf, welches dazu ausgebildet ist, einen Winkel zwischen der Dentalspule und der ersten Fläche und/oder der Patientenlagerungsvorrichtung einzustellen. Vorzugsweise kann die Dentalspule mittels des Scharniers oder des Gelenks gegenüber der ersten Fläche und/oder der Patientenlagerungsvorrichtung verkippt werden. Das Scharnier oder das Gelenk kann ferner einen Mechanismus aufweisen, welcher dazu ausgebildet ist, die Dentalspule in einem vorbestimmten Winkel zu der ersten Fläche zu arretieren. Es ist vorstellbar, dass eine Arretierung der Dentalspule in dem vorbestimmten Winkel zu der ersten Fläche mittels eines Rastmechanismus, eines Führungsmechanismus, eines Steckmechanismus oder eines Klemmmechanismus implementiert ist. Daneben sind selbstverständlich beliebige weitere kraftschlüssige und/oder formschlüssige Arretierungsmechanismen vorstellbar. In einer weiteren Ausführungsform ist die Positionierungseinheit mittels des Schwenkmechanismus schwenkbar gelagert, wobei ein Winkel der Positionierungseinheit gegenüber einer Magnetresonanzvorrichtung und/oder einem Patienten einstellbar ist.

Durch das Bereitstellen einer erfindungsgemäßen Positionierungseinheit lässt sich eine Position und/oder eine Orientierung der Dentalspule auf besonders zeiteffiziente Weise relativ zu dem Patienten verändern. Weiterhin kann ein Blockieren eines Zugangs des Patienten zu der Patientenlagerungsfläche durch eine positionierbare Dentalspule vorteilhaft vermieden werden.

In einer bevorzugten Ausführungsform der erfindungsgemäßen Dentalspule weist die Halterung einen Schwenkmechanismus auf, wobei das erste Element und/oder das zweite Element bei anwendungsgemäßer Positionierung der Dentalspule an der Kieferregion des Patienten mittels des Schwenkmechanismus um einen maximalen Winkel schwenkbar gegenüber dem Patienten gelagert sind und wobei der maximale Winkel des ersten Elements und/oder des zweiten Elements gegenüber dem Patienten so gewählt ist, dass ein Kopf des Patienten ungehindert in einer anwendungsgemäßen Relativposition zu der Halterung positioniert werden kann.

Der Schwenkmechanismus kann beispielsweise ein Scharnier, ein Gelenk, ein Drehlager, ein Gleitlager, ein Wälzlager, ein Rollenlager und/oder einen beliebigen anderen Mechanismus aufweisen, welcher dazu ausgebildet ist, das erste Element und/oder das zweite Element in einem Winkel gegenüber der Halterung und/oder der Patientenlagerungsvorrichtung zu schwenken. Vorzugsweise sind das erste Element und/oder das zweite Element bei anwendungsgemäßer Positionierung der Dentalspule entlang einer Sagittalebene des Patienten schwenkbar. Es ist aber ebenso vorstellbar, dass das erste Element und/oder das zweite Element annähernd parallel zu einer Frontalebene des Patienten schwenkbar sind. Die Dentalspule kann beim Schwenken einem Abschnitt eines Kreisbogens folgen, welcher durch den Schwenkmechanismus bestimmt ist. Es ist vorstellbar, dass der Schwenkmechanismus gemäß einer oben beschriebenen Ausführungsform von der Positionierungseinheit umfasst und/oder in diese integriert ist.

In einer bevorzugten Ausführungsform weist der Schwenkmechanismus eine Rasterung oder einen Widerstand auf, welche ein Erreichen eines vorbestimmten Winkels markieren und/oder ein ungewünschtes bzw. unkontrolliertes Schwenken verhindern. Es ist ferner vorstellbar, dass der Schwenkmechanismus eine Anordnung von Nocken oder Nippeln umfasst, welche einer Schwenkbewegung des ersten Elements und/oder des zweiten Elements einen Widerstand entgegensetzen und ein unkontrolliertes Zuklappen oder Aufklappen der Dentalspule verhindern.

Der maximale Winkel kann in Abhängigkeit eines Aufbaus der Magnetresonanzvorrichtung und/oder der Patientenlagerungsvorrichtung zwischen 60° und 90°, 90° und 180° oder 180° und 270° betragen. Bei Magnetresonanzvorrichtungen, bei welchen der Patienten für die Magnetresonanzuntersuchung eine stehende oder sitzende Körperhaltung einnimmt, beträgt der maximale Winkel vorzugsweise zwischen 60° und 90°. Es sind aber auch größere Werte des maximalen Winkels vorstellbar. Bei konventionellen Magnetresonanzvorrichtungen mit einem Patiententisch ist der maximale Winkel vorzugsweise kleiner als 180°.

Durch das Bereitstellen eines Schwenkmechanismus lassen sich das erste Element und/oder das zweite Element auf besonders zeiteffiziente Weise in einem geringen Abstand zu der Kieferregion des Patienten positionieren und wieder vollständig aus einem Zugangsbereich des Patienten zu der Patientenlagerungsvorrichtung entfernen. Dadurch lässt sich ein erhöhter Zeitaufwand, welcher mit einer anwendungsgemäßen Positionierung der Dentalspule an der Kieferregion verbunden sein kann, auf vorteilhafte Weise kompensieren.

In der erfindungsgemäßen Dentalspule weist die Halterung einen Führungsmechanismus mit einer Führungsachse auf, wobei die Führungsachse im Wesentlichen parallel zu einer Schnittlinie einer Sagittalebene und einer Transversalebene an der Kieferregion des Patienten ausgerichtet ist und wobei der Führungsmechanismus dazu ausgebildet ist, das erste Element entlang der Führungsachse zu positionieren, um einen Abstand zwischen dem ersten Element und der Kieferregion des Patienten einzustellen.

Gemäss der Erfindung ist die Führungsachse im Wesentlichen parallel zu der Schnittlinie einer Sagittalebene und einer Transversalebene an der Kieferregion des Patienten ausgerichtet. Es ist jedoch ebenso vorstellbar, jedoch ausserhalb des durch die Patentansprüche definierten Schutzumfangs, dass eine Ausrichtung der Führungsachse um einen Winkel, z. B. einen Winkel zwischen 0 und 15° oder zwischen 15 und 30°, gegenüber der Transversalebene verkippt ist. In besonderen Fällen kann die Führungsachse auch um wenige Grad gegenüber der Sagittalebene verkippt sein.

Der Führungsmechanismus kann ein Führungselement, wie z. B. einen Zylinder oder eine Schraube, aufweisen. Beispielsweise kann die Schraube in ein Gewinde des ersten Elements und/oder des zweiten Elements eingreifen. Ein Drehen der Schraube kann ein Einschrauben der Schrauben in das Gewinde des ersten Elements und/oder des zweiten Elements bewirken, welche dadurch in Richtung der Schraube bewegt werden. Die Schraube kann hierfür in einer vorbestimmten Position zu der Halterung gelagert sein, sodass eine Bewegung der Schraube in Richtung des ersten Elements und/oder des zweiten Elements bei einem Drehen vermieden wird. Die Halterung kann insbesondere einen oder mehrere Stifte oder Bolzen aufweisen, welche in komplementäre Aussparungen oder Löcher in dem ersten Element und/oder dem zweiten Element eingreifen. Die Stifte oder Bolzen sind vorzugsweise dazu ausgelegt, eine Führung des ersten Elements und/oder des zweiten Elements entlang einer Achse der Stifte oder Bolzen bereitzustellen, wenn das erste Element und/oder das zweite Element mittels des Führungsmechanismus entlang der Führungsachse positioniert werden. Die Stifte oder Bolzen sind vorzugsweise parallel zu der Führungsachse orientiert.

Es ist ferner vorstellbar, dass das erste Element und/oder das zweite Element mechanisch mit einem Halteelement verbunden sind. Die Schraube des Führungsmechanismus kann entsprechend in ein Gewinde des Halteelements eingreifen, um das erste Element und/oder das zweite Element entlang der Führungsachse des Führungsmechanismus zu positionieren. Das Halteelement kann ferner eine oder mehrere Aussparungen oder Löcher aufweisen, welche komplementär zu den Stiften oder Bolzen der Halterung ausgebildet sind. Die Aussparungen oder Löcher können entsprechend dem oben beschriebenen Beispiel eine Führung für die Stifte oder Bolzen bereitstellen, welche ein Verdrehen oder Verkippen des Halteelements in Relation zu der Führungsachse vermeiden.

Die Führungsachse kann insbesondere eine Rotationsachse der Schraube des Führungsmechanismus darstellen. Vorzugsweise ist die Führungsachse parallel zu einer Sagittalebene, insbesondere einer medianen oder mittleren Sagittalebene, des Patienten ausgerichtet.

Mittels des erfindungsgemäßen Führungsmechanismus lässt sich eine Feinjustierung der Position des ersten Elements und/oder des zweiten Elements an der Kieferregion des Patienten vornehmen. Dadurch lässt sich ein besonders geringer Abstand zwischen der Hautoberfläche der Kieferregion und des ersten Elements und/oder des zweiten Elements bereitstellen, wodurch das Signal-zu-Rausch Verhältnis der Dentalspule vorteilhaft verbessert werden kann. Es ist ebenso vorstellbar, dass das erste Element und das zweite Element (aber auch das dritte Element) mittels des Führungsmechanismus entlang der Führungsachse besonders gleichmäßig auf der Hautoberfläche der Kieferregion des Patienten positioniert werden können. Dadurch lässt sich insbesondere eine Verdrehung oder Verkippung des ersten Elements und/oder des zweiten Elements vermeiden, welche zu einer ungleichmäßigen Anordnung von Antennen des ersten Elements und des zweiten Elements an der Kieferregion führen kann.

In einer Ausführungsform weist die Halterung der erfindungsgemäßen Dentalspule eine Schwanenhalsmechanik oder einen Gelenkschlauch auf.

Eine Schwanenhalsmechanik kann einen biegsamen, gewendelten Tubus aufweisen, welche in eine beliebige Raumrichtung gebogen werden kann und dazu ausgebildet ist, eine gebogene Form aufrechtzuerhalten. Hierfür kann die Schwanenhalsmechanik einen schraubenförmigen, inneren Draht aufweisen, welcher von einem Draht mit einem im Wesentlichen dreikantigen Querschnitt umwickelt ist.

Ein Gelenkschlauch kann z. B. aus kugelgelenkartigen Formstücken zusammengesetzt sein. Die kugelgelenkartigen Formstücke können miteinander verklemmt sein und eine vorbestimmte Haltekraft aufweisen, welche eine Gewichtskraft des ersten Elements und/oder des zweiten Elements überschreitet. Der Gelenkschlauch kann zur Positionierung des ersten Elements und/oder des zweiten Elements (sowie des dritten Elements) manuell verformt oder gebogen werden und eine Form für die Dauer einer Magnetresonanzmessung aufrechterhalten.

Es ist ferner vorstellbar, dass die Schwanenhalsmechanik oder der Gelenkschlauch einen Arretierungsmechanismus aufweisen, welcher dazu ausgebildet ist, die Schwanenhalsmechanik oder den Gelenkschlauch in einer vorbestimmten Form zu arretieren oder festzustellen. Es ist weiterhin vorstellbar, dass die Schwanenhalsmechanik oder der Gelenkschlauch mechanisch mit einem Halteelement verbunden sind, welches das erste Element und/oder das zweite Element trägt und/oder einer vorbestimmten Relativposition hält.

Durch das Bereitstellen einer Schwanenhalsmechanik oder eines Gelenkschlauchs lassen sich das erste Element und/oder das zweite Element auf besonders einfache und zeiteffiziente Weise relativ zu der Kieferregion des Patienten positionieren. Ferner können eine Schwanenhalsmechanik und ein Gelenkschlauch eine besonders kostengünstige Lösung für die Positionierung der Dentalspule darstellen und einen Fertigungsaufwand der Dentalspule vorteilhaft reduzieren. Weiterhin bieten eine Schwanenhalsmechanik oder ein Gelenkschlauch eine erhöhte Offenheit der Dentalspule. Dadurch kann der Komfort des Patienten erhöht und ein Risiko eines Abbruchs einer Magnetresonanzmessung minimiert werden.

Gemäß einer weiteren Ausführungsform der erfindungsgemäßen Dentalspule weist der Schwenkmechanismus ferner ein Dämpfungselement auf, welches dazu ausgebildet ist, eine Bewegung des ersten Elements und/oder des zweiten Elements bei Schwenkung des ersten Elements und/oder des zweiten Elements entlang einer vorbestimmten Wegstrecke abzudämpfen und/oder zu begrenzen.

In einer bevorzugten Ausführungsform ist das Dämpfungselement als eine mechanische Feder, ein pneumatisches Element, wie z. B. eine Gasfeder, oder ein elastisches Element ausgeführt. Das Dämpfungselement kann dabei entlang der vorbestimmten Wegstrecke des ersten Elements und/oder des zweiten Elements ausgerichtet sein. Beispielsweise kann die Feder so positioniert sein, dass eine Bewegung des ersten Elements und/oder des zweiten Elements umso stärker gebremst und/oder gedämpft wird, je weiter das erste Element und/oder das zweite Element entlang der vorbestimmten Wegstrecke in Richtung der Kieferregion des Patienten geschwenkt werden.

Es ist aber ebenso vorstellbar, dass das Dämpfungselement gemäß einer oben beschriebenen Ausführungsform, eine Anordnung von Nocken, Rastelementen oder Nippeln aufweist, welche dazu ausgebildet sind, einer Bewegung des ersten Elements und/oder des zweiten Elements entlang der vorbestimmten Wegstrecke einen Widerstand entgegenzusetzen. Das Dämpfungselement kann ferner als ein Bremselement ausgebildet sein, welches eine Bewegung des ersten Elements und/oder des zweiten Elements mittels einer kraftschlüssigen Verbindung mit der Halterung bremst oder begrenzt. Ein Widerstand, welcher der Bewegung des ersten Elements und/oder des zweiten Elements entlang der vorbestimmten Wegstrecke mittels des Dämpfungselements entgegengesetzt wird, kann dabei mit einer Distanz zwischen dem ersten Element und/oder dem zweiten Element und der Kieferregion variieren und in Richtung der Kieferregion des Patienten zunehmen.

Durch Bereitstellen eines erfindungsgemäßen Dämpfungselements lässt sich ein unkontrolliertes Einklappen oder Schwenken der Dentalspule auf vorteilhafte Weise vermeiden oder begrenzen. Dies kann insbesondere bei Magnetresonanzvorrichtungen, bei denen der Patient in einer vertikalen oder stehenden Position ausgerichtet ist, einer Verletzung des Patienten infolge einer unsachgemäßen Positionierung der Dentalspule durch einen Bediener oder den Patienten vorbeugen.

In einer weiteren Ausführungsform umfasst die erfindungsgemäße Dentalspule ferner ein flexibles Halteelement, welches bei anwendungsgemäßer Positionierung der Dentalspule an der Kieferregion des Patienten zumindest mit dem zweiten Element verbunden ist, wobei der Anpassungsmechanismus als ein Zugmechanismus ausgestaltet ist, welcher mechanisch mit dem flexiblen Halteelement verbunden ist und dazu ausgelegt ist, eine Zugkraft bereitzustellen, welche das flexible Halteelement in eine Richtung der Kieferregion des Patienten führt.

Das erste Element und/oder das zweite Element können mit dem flexiblen Halteelement verbunden sein und/oder von diesem gehalten werden. Das erste Element und/oder das zweite Element können insbesondere kraftschlüssig, formschlüssig und/oder stoffschlüssig mit dem flexiblen Halteelement verbunden sein. In einer bevorzugten Ausführungsform ist das flexible Halteelement relativ zu einem Trägerelement und/oder einer Positionierungseinheit der Halterung positionierbar und hält zumindest das zweite Element in einer anwendungsgemäßen Position an der Kieferregion des Patienten. Der Zugmechanismus kann insbesondere ein Anpassungsmechanismus gemäß einer oben beschriebenen Ausführungsform darstellen.

Es ist insbesondere vorstellbar, dass das flexible Halteelement bei anwendungsgemäßer Positionierung der Dentalspule zwischen der Kieferregion des Patienten und der Halterung positioniert ist. Das flexible Halteelement kann dabei eine im Wesentlichen quaderförmige oder zu einem Quader homöomorphe Gestalt aufweisen, welche die Kieferregion von einer linken Wangenregion bis zu einer rechten Wangenregion des Patienten umlaufend umschließt. Vorzugsweise ist ein erstes Ende des flexiblen Halteelements an der linken Wangenregion und ein zweites Ende des flexiblen Halteelements an der rechten Wangenregion mechanisch mit dem Zugmechanismus verbunden. Der Zugmechanismus kann ein Zugelement, wie z. B. ein Band, ein Seil, ein Netz, eine Schnur oder dergleichen, aufweisen, welches das erste Ende und das zweite Ende mittels des Zugmechanismus in eine von dem Patienten abgewandte Richtung führt oder auslenkt. Eine solche Richtung kann im Wesentlichen von einer dorsalen Seite des Patienten aus in eine dem Patienten abgewandte Richtung weisen. Der Zugmechanismus kann hierbei einen beliebigen Mechanismus aufweisen, welcher dazu ausgebildet ist, eine Zugkraft auf das Zugelement auszuüben.

In einer Ausführungsform ist das flexible Halteelement mittels des Zugmechanismus an die Kieferregion des Patienten anformbar. Dies kann bedeuten, dass das flexible Halteelement mittels des Zugmechanismus entlang der Kieferregion des Patienten positionierbar ist. Das flexible Halteelement kann beispielsweise auf der Hautoberfläche des Patienten aufliegen und mittels der Zugkraft an diese angeformt werden. Es ist aber ebenso vorstellbar, dass das flexible Halteelement mittels des ersten Elements und/oder des zweiten Elements von der Hautoberfläche des Patienten beabstandet ist. In diesem Fall kann das flexible Halteelement dazu ausgebildet sein, zumindest das zweite Element bei Ausübung einer Zugkraft auf das Zugelement elastisch zu verformen und der Kieferregion des Patienten nachzuformen.

In einer alternativen Ausführungsform ist das flexible Halteelement dazu ausgebildet, relativ zu der Halterung positioniert und unter elastischer Verformung in die Halterung eingespannt zu werden. Das flexible Halteelement kann das zweite Element dabei unabhängig von dem Anpassungsmechanismus an die Kieferregion des Patienten anformen. Beispielsweise können elastische Rückstellkräfte des flexiblen Halteelements so gewählt sein, dass das flexible Halteelement eine kraftschlüssige Verbindung mit der Halterung ausbildet. Es ist insbesondere vorstellbar, dass das flexible Halteelement bei anwendungsgemäßer Positionierung zwischen der Kieferregion des Patienten und der Halterung positioniert und/oder eingespannt ist. Das flexible Halteelement kann dabei an die Kieferregion des Patienten angeformt sein. Die elastische Rückstellkraft des flexiblen Halteelements kann insbesondere auf eine vorbestimmte Gruppe von Patienten, wie z. B. Kinder, Jugendliche, Erwachsene oder Senioren, abgestimmt sein, sodass das flexible Halteelement bei anwendungsgemäßer Positionierung an der Kieferregion des Patienten einen vorbestimmten Druck auf die Kieferregion des Patienten und/oder das zweite Element ausübt. Wie oben beschrieben, kann zumindest das zweite Element zwischen der Hautoberfläche der Kieferregion des Patienten und dem flexiblen Halteelement positioniert sein und bei Positionierung des flexiblen Halteelements der Kieferregion des Patienten nachgeformt werden.

Durch Bereitstellen eines erfindungsgemäßen flexiblen Halteelements lässt sich das zweite Element auf besonders zeiteffiziente Weise vorteilhaft der Kieferregion des Patienten nachformen.

In einer Ausführungsform weist die erfindungsgemäße Lokalspule ferner ein drittes Element mit einem flexiblen Element auf, wobei das flexible Element dazu ausgebildet ist, ein Nachformen des dritten Elements der Kieferregion des Patienten zu ermöglichen und wobei das dritte Element bei anwendungsgemäßer Positionierung der Dentalspule auf einer dem zweiten Element gegenüberliegenden Seite der Kieferregion des Patienten angeordnet ist, wobei das dritte Element eine Antenne aufweist, welche dazu ausgebildet ist, hochfrequente Signale in einem Frequenz- und Leistungsbereich einer Magnetresonanzmessung zu empfangen.

Das dritte Element kann gemäß einer oben beschriebenen Ausführungsform ausgestaltet sein. Das dritte Element kann insbesondere als ein Teil des zweiten Elements ausgebildet sein, mechanisch mit dem zweiten Element verbunden sein oder getrennt von dem zweiten Element vorliegen. Das dritte Element teilt die Vorteile des zweiten Elements. Insbesondere lässt sich das Nachformen der Kieferregion mittels der Dentalspule durch mittels eines separaten dritten Elements weiter verbessern.

In einer weiteren Ausführungsform ist der Anpassungsmechanismus dazu ausgebildet, das erste Element und das zweite Element relativ zueinander zu positionieren.

Hierfür können das erste Element und/oder das zweite Element mechanisch mit getrennten Trägerelementen der Halterung verbunden sein. Die getrennten Trägerelemente können ein reversibel verformbares Material und/oder einen Anpassungsmechanismus aufweisen, welche dazu ausgebildet sind, das zweite Element relativ zu dem ersten Element zu positionieren. In analoger Weise kann das dritte Element mittels eines getrennten Trägerelements der Halterung gegenüber dem zweiten Element positionierbar sein.

Es ist jedoch ebenso vorstellbar, dass das erste Element und das zweite Element mechanisch mit einem Halteelement der Halterung, wie z. B. dem flexiblen Halteelement, verbunden sind. Das Halteelement kann dabei ein plastisch oder elastisch verformbares Material aufweisen. Bei einem Verformen des Halteelements kann das erste Element entsprechend relativ gegenüber dem zweiten Element positioniert werden.

Durch Bereitstellen einer Halterung, welche dazu ausgebildet ist, das erste Element, das zweite Element und/oder das dritte Element relativ zueinander zu positionieren, lassen sich die Antenne oder die Antennen der Dentalspule auf vorteilhafte Weise einer individuellen Geometrie der Kieferregion des Patienten nachformen.

Gemäß einer Ausführungsform weist die erfindungsgemäße Dentalspule ein Fixierungselement auf, welches dazu ausgebildet ist, die Halterung und/oder das erste Element mechanisch mit einem Kopf des Patienten zu verbinden, um den Kopf des Patienten in einer vorbestimmten Relativposition zu dem ersten Element zu fixieren.

Das Fixierungselement kann beispielsweise als eine schlingenförmige Schnur, ein Band oder dergleichen ausgebildet sein, welches mechanisch mit der Halterung und/oder dem ersten Element verbunden ist und ein Kinn, einen Unterkiefer und/oder einen Hinterkopf des Patienten umgreift. Die Schnur oder das Band kann insbesondere dazu ausgebildet sein, das erste Element und/oder die Halterung an dem Kopf des Patienten zu befestigen und/oder gegen das Kinn bzw. den Unterkiefer zu verspannen. In einer Ausführungsform kann das Fixierungselement mit einem Zugmechanismus gemäß einer oben beschriebenen Ausführungsform verbunden sein, welcher dazu ausgelegt ist, das Fixierungselement mit einer vorbestimmten Zugkraft zu beaufschlagen, sodass das erste Element und/oder die Halterung mit einer der vorbestimmten Zugkraft entsprechenden Kraft auf die Hautoberfläche der Kieferregion des Patienten angedrückt werden.

In einer weiteren Ausführungsform kann das Fixierungselement ein elastisches Material, wie z. B. ein Elastomer, insbesondere ein Gummiband oder einen elastischen Schaumstoff, aufweisen. Beispielsweise kann das Fixierungselement, wie oben beschrieben, bei einer Positionierung zwischen der Halterung und/oder dem ersten Element und der Kieferregion des Patienten elastisch verformt werden, wobei elastische Rückstellkräfte des Fixierungselements die Kieferregion und/oder den Kopf des Patienten relativ zu der Halterung und/oder dem ersten Element fixieren. Insbesondere kann das Fixierungselement als ein elastischer Schaumstoff ausgebildet sein. Der elastische Schaumstoff kann auch zwischen der Halterung und dem zweiten Element eingespannt sein und das zweite Element auf die Hautoberfläche des Patienten drücken, sodass der Kopf des Patienten in einer vorbestimmten Position zu dem ersten Element fixiert wird.

In einer weiteren Ausführungsform ist das Fixierungselement mechanisch mit dem ersten Element verbunden und mittels eines geeigneten Mechanismus (z. B. des Führungsmechanismus) auf der Hautoberfläche der Kieferregion des Patienten positionierbar. Das Fixierungselement kann hierbei insbesondere ein elastisches Material, wie z. B. ein mit Fluid (z. B. Luft, Inertgas oder einer Flüssigkeit) befülltes Kissen, einen Schaumstoff, ein Elastomer oder dergleichen, umfassen, welches auf einer der Kieferregion des Patienten zugewandten Seite des ersten Elements positioniert ist. Das Fixierungselement kann insbesondere dazu ausgelegt sein, einen Druck auf die Hautoberfläche der Kieferregion des Patienten zu verteilen und/oder abzupuffern. Vorzugsweise ist das erste Element mittels eines Führungsmechanismus gemäß einer oben beschriebenen Ausführungsform auf der Hautoberfläche des Patienten positionierbar.

In einer Ausführungsform ist das Fixierungselement dazu ausgebildet, zwischen einer Stirn des Patienten und der Halterung verspannt zu werden und eine kraftschlüssige Verbindung mit der Halterung mit der Stirn des Patienten bereitzustellen.

Durch eine Fixierung der Halterung an der Stirn des Patienten lässt sich auf vorteilhafte Weise eine robuste und reproduzierbare Positionierung der Dentalspule an der Kieferregion des Patienten bereitstellen.

Mittels des erfindungsgemäßen Fixierungselements lässt sich die erfindungsgemäße Dentalspule auf vorteilhafte Weise in einer konsistenten oder beständigen Relativposition zu der Kieferregion des Patienten positionieren.

In einer weiteren Ausführungsform weist die erfindungsgemäße Dentalspule ferner einen Sicherheitsmechanismus auf, welcher dazu ausgelegt ist, die Dentalspule bei einer Betätigung des Sicherheitsmechanismus durch eine Person in eine Öffnungsposition zu überführen, um die Dentalspule aus der anwendungsgemäßen Position an der Kieferregion des Patienten zu entfernen.

Der Sicherheitsmechanismus kann beispielsweise als eine Reißleine ausgeführt sein, welche dazu ausgebildet ist, die Dentalspule, die Halterung, das erste Element und/oder das zweite Element bei Ausübung einer vorbestimmten Zugkraft aus der anwendungsgemäßen Position an der Kieferregion des Patienten zu entfernen. Vorzugsweise ist die Reißleine mechanisch mit dem ersten Element und/oder dem zweiten Element verbunden. Es ist aber ebenso vorstellbar, dass die Reißleine mechanisch mit einem Sicherungselement und/oder dem Halteelement verbunden ist, welches das erste Element und/oder das zweite Element in einer vorbestimmten Relativposition mit der Halterung verbindet. Ein Lösen des Sicherungselements und/oder des Halteelements mittels eines Zugs an der Reißleine kann das erste Element und/oder das zweite Element von der Halterung lösen oder freigeben.

Es jedoch ebenso vorstellbar, dass der Sicherheitsmechanismus einen elektronischen Mechanismus und ein Stellelement aufweist. Der elektronische Mechanismus kann einen Schalter, einen Knopf, eine Kamera und/oder ein Mikrofon aufweisen, welche dazu ausgebildet sind, einen Schaltbefehl, eine Geste und/oder eine Sprachnachricht des Patienten und/oder des Bedieners zu erfassen. Die Geste und/oder die Sprachnachricht können insbesondere einen Befehl des Patienten oder Bedieners zur Überführung der Dentalspule in die Öffnungsposition kodieren. Der Sicherheitsmechanismus kann ferner eine Recheneinheit, eine Steuereinheit und/oder eine Logikeinheit aufweisen, welche in Abhängigkeit des Schaltbefehls, der Geste und/oder der Sprachnachricht ein Steuersignal zur Positionierung des Stellelements ausgeben. Das Steuersignal kann insbesondere an einen Antrieb übertragen werden, welcher dazu ausgebildet ist, eine Position des Stellelements zu verändern. Ein geeigneter Antrieb kann insbesondere ein elektrischer Antrieb, aber auch ein pneumatischer, ein hydraulischer, ein motorischer oder ein manueller Antrieb, sein. Vorzugsweise ist das Stellelement mechanisch mit dem Sicherungselement oder und/oder dem Haltelement verbunden, welches das erste Element und/oder das zweite Element in der vorbestimmten Relativposition mit der Halterung verbindet. Auf diese Weise kann der Sicherheitsmechanismus das erste Element und/oder das zweite Element in Abhängigkeit des Schaltbefehls, der Geste und/oder der Sprachnachricht von der Halterung lösen und die Dentalspule in eine Öffnungsposition überführen.

Durch Bereitstellen eines erfindungsgemäßen Sicherheitsmechanismus lässt sich die Dentalspule bei Auftreten eines Unwohlseins des Patienten und/oder bei Überschreiten eines tolerierbaren Drucks auf die Hautoberfläche der Kieferregion auf vorteilhafte Weise in eine Öffnungsposition überführen. Dadurch lassen sich Unfälle bei der Positionierung der erfindungsgemäßen Dentalspule vermeiden und ein Sicherheitsgefühl oder Vertrauen des Patienten bei einer Magnetresonanzmessung mit der Dentalspule auf vorteilhafte Weise erhöhen.

Gemäß einer Ausführungsform weist die erfindungsgemäße Dentalspule eine Sendeeinheit und eine Empfangseinheit auf, wobei die Sendeeinheit zumindest eine Antenne aufweist, welche dazu ausgelegt ist, hochfrequente Signale in einem Frequenz- und Leistungsbereich einer Magnetresonanzvorrichtung in eine Kieferregion eines Patienten auszusenden und wobei die Empfangseinheit zumindest eine Antenne aufweist, welche dazu ausgebildet ist, Magnetresonanzsignale aus der Kieferregion des Patienten zu empfangen. Es ist vorstellbar, dass die zumindest eine Antenne der Sendeeinheit mit der zumindest einen Antenne der Empfangseinheit übereinstimmt.

Durch das Bereitstellen einer Dentalspule mit einer Sendeeinheit kann die zumindest eine Antenne in unmittelbarer Nähe zu der Kieferregion des Patienten positioniert werden. Dadurch lässt sich eine Anregung eines Gewebes des Patienten vorteilhaft auf die Kieferregion beschränken, wodurch sich eine Effizienz der Dentalspule erhöhen lässt.

Die erfindungsgemäße Magnetresonanzvorrichtung umfasst eine Dentalspule gemäß einer oben beschriebenen Ausführungsform, wobei die Magnetresonanzvorrichtung dazu ausgebildet ist, hochfrequente Signale in einem Frequenz- und Leistungsbereich einer Magnetresonanzmessung aus einer Kieferregion eines Patienten mittels der Dentalspule zu erfassen, um eine Bildgebung der Kieferregion des Patienten durchzuführen.

Die Magnetresonanzvorrichtung kann eine Halterung gemäß einer oben beschriebenen Ausführungsform aufweisen. Die Halterung kann dazu ausgebildet sein, zumindest eine Komponente der Dentalspule, wie z. B. ein erstes Element, ein zweites Element, ein drittes Element und/oder ein Halteelement, in einer vorbestimmten Relativposition zu einer Kieferregion des Patienten zu halten und/oder zu positionieren. Die Halterung kann ferner mechanisch mit der Magnetresonanzvorrichtung, insbesondere einer Patientenlagerungsvorrichtung und/oder einem Patiententisch, verbunden sein. Es ist aber ebenso vorstellbar, dass die Halterung eine von der Magnetresonanzvorrichtung getrennte Komponente ist. Die Halterung kann in diesem Fall an einer Wand und/oder einer Decke eines Untersuchungsraums der Magnetresonanzvorrichtung montiert sein oder reversibel an dem Patiententisch angebracht sein. Vorzugsweise weist die Halterung eine Positionierungseinheit auf, welche dazu ausgebildet ist, eine Position der Halterung und/oder der zumindest einen Komponente der Dentalspule relativ zu der Patientenlagerungsvorrichtung und/oder dem Patienten einzustellen. Es ist ferner vorstellbar, dass die Positionierungseinheit dazu ausgelegt ist, eine räumliche Position und/oder Orientierung der zumindest einen Komponente der Dentalspule und/oder der Halterung einzustellen.

Ferner weist die erfindungsgemäße Magnetresonanzvorrichtung zumindest eine elektrische Anschlussleitung auf, welche dazu ausgelegt ist, eine Antenne der Dentalspule elektrisch mit der Magnetresonanzvorrichtung zu verbinden. In einer Ausführungsform weist die Dentalspule eine oder mehrere Antennen auf, welche als eine Sendeeinheit ausgebildet sind. Die Sendeeinheit kann mittels einer elektrischen Anschlussleitung mit einer Hochfrequenzeinheit der Magnetresonanzvorrichtung verbunden sein. Es ist vorstellbar, dass die Hochfrequenzeinheit einen Wechselstrom bereitstellt, welcher als hochfrequentes Signal von der Sendeeinheit in ein Volumen der Kieferregion des Patienten ausgesendet wird, sodass ein B1-Magnetfeld erzeugt wird. In einer weiteren Ausführungsform weist die Dentalspule eine oder mehrere Antennen auf, welche als eine Empfangseinheit ausgebildet sind. Die Empfangseinheit kann mittels einer elektrischen Anschlussleitung mit einem Empfängerkanal der Magnetresonanzvorrichtung verbunden sein. Die Magnetresonanzvorrichtung ist somit in der Lage, Magnetresonanzsignale der Kieferregion des Patienten zu empfangen und Magnetresonanzbilder in Abhängigkeit der empfangenen Magnetresonanzsignale zu rekonstruieren.

Durch die erfindungsgemäße Magnetresonanzvorrichtung kann auf vorteilhafte Weise eine zeiteffiziente und wiederholbare Aufnahme von Magnetresonanzbildern der Kieferregion des Patienten ermöglicht werden. Weiterhin teilt die erfindungsgemäße Magnetresonanzvorrichtung die Vorteile der erfindungsgemäßen Dentalspule gemäß einer oben beschriebenen Ausführungsform.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: eine schematische Darstellung einer Ausführungsform einer erfindungsgemäßen Magnetresonanzvorrichtung,
- Fig. 2: eine schematische Darstellung einer Ausführungsform einer erfindungsgemäßen Dentalspule,
- Fig. 3: eine mögliche Ausführungsform einer erfindungsgemä-ßen Dentalspule,
- Fig. 4: eine mögliche Ausführungsform einer erfindungsgemä-ßen Dentalspule,
- Fig. 5: eine mögliche Ausführungsform einer erfindungsgemä-ßen Dentalspule,
- Fig. 6: eine mögliche Ausführungsform einer erfindungsgemä-ßen Dentalspule,
- Fig. 7: eine mögliche Ausführungsform einer erfindungsgemä-ßen Dentalspule,
- Fig. 8: eine schematische Darstellung einer Ausführungsform einer erfindungsgemäßen Dentalspule,
- Fig. 9: eine mögliche Ausführungsform einer Halterung einer erfindungsgemäßen Dentalspule.
- Fig. 10: eine mögliche Ausführungsform einer Halterung einer erfindungsgemäßen Dentalspule,
- Fig. 11: eine mögliche Ausführungsform einer erfindungsgemä-ßen Dentalspule.

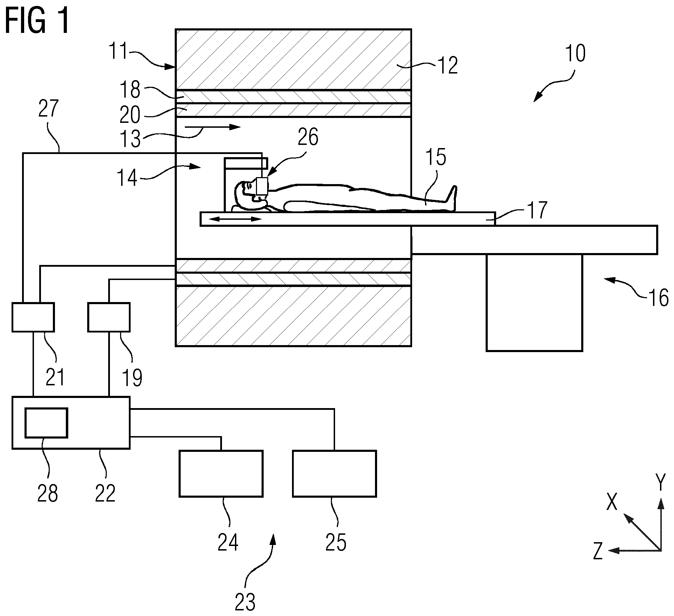

In Fig. 1 ist eine mögliche Ausführungsform einer erfindungsgemäßen Magnetresonanzvorrichtung 10 mit einer erfindungsgemäßen Dentalspule 26 schematisch dargestellt. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Grundmagnetfelds 13 (B0-Magnetfeld) aufweist. Zudem umfasst die Magnetresonanzvorrichtung 10 einen Patientenaufnahmebereich 14 zu einer Aufnahme eines Patienten 15. Der Patientenaufnahmebereich 14 ist in dem vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung während einer Magnetresonanzmessung verwendet wird. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Atomkernen ausgelegt, die sich in dem von dem Hauptmagneten 12 erzeugten Grundmagnetfeld 13 befinden. Die Körperspule 20 wird von einer Hochfrequenzeinheit 21 der Magnetresonanzvorrichtung 10 angesteuert und strahlt hochfrequente Signale in einen Untersuchungsraum ein, der im Wesentlichen von einem Patientenaufnahmebereich 14 der Magnetresonanzvorrichtung 10 gebildet ist. Die Körperspule 20 kann weiterhin auch zu einem Empfangen von Magnetresonanzsignalen ausgebildet sein.

Für eine Steuerung des Hauptmagneten 12, der Gradientensteuereinheit 19 und der Hochfrequenzeinheit 21 weist die Magnetresonanzvorrichtung 10 eine Steuereinheit 22 auf. Die Steuereinheit 22 ist dazu ausgebildet eine Durchführung einer Sequenz, wie z. B. einer bildgebenden Gradientenechosequenz, einer TSE-Sequenz oder einer UTE-Sequenz, zu steuern. Zudem umfasst die Steuereinheit 22 eine Auswerteeinheit 28 zu einer Auswertung von digitalisierten Magnetresonanzsignalen, die während der Magnetresonanzmessung erfasst werden.

Des Weiteren umfasst die Magnetresonanzvorrichtung 10 eine Benutzerschnittstelle 23, welche eine Signalverbindung mit der Steuereinheit 22 aufweist. Steuerinformationen, wie beispielsweise Bildgebungsparameter und rekonstruierte Magnetresonanzbilder, können auf einer Anzeigeeinheit 24, beispielsweise auf zumindest einem Monitor, der Benutzerschnittstelle 23 für einen Nutzer angezeigt werden. Weiterhin weist die Benutzerschnittstelle 23 eine Eingabeeinheit 25 auf, mittels der Parameter einer Magnetresonanzbildgebung von dem Nutzer eingegeben werden können.

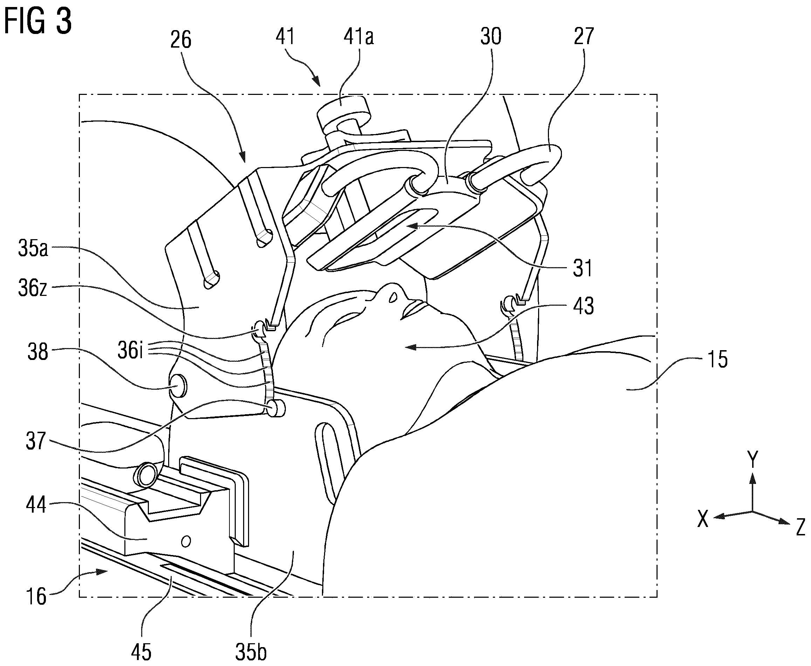

Ferner weist die Magnetresonanzvorrichtung 10 eine Dentalspule 26 auf, welche an der Kieferregion 43 eines Patienten 15 positioniert ist (siehe Fig. 3) und Magnetresonanzsignale aus einem Volumen der Kieferregion 43 an die Magnetresonanzvorrichtung 10 überträgt. Die Dentalspule 26 weist vorzugsweise eine elektrische Anschlussleitung 27 auf, welche eine Signalverbindung mit der Hochfrequenzeinheit 21 und der Steuereinheit 22 bereitstellt. Ebenso wie die Körperspule 20 kann auch die Dentalspule 26 zu einer Anregung von Atomkernen und zu einem Empfangen von Magnetresonanzsignalen ausgebildet sein. Zum Aussenden von hochfrequenten Signalen wird eine Sendeeinheit der Dentalspule 26 von der Hochfrequenzeinheit 21 angesteuert. Die Dentalspule 26 kann den Kopf des Patienten 15 außenumfänglich entlang einer Längsachse des Patienten 15 umschließen. Die Sendeeinheit und/oder eine Empfangseinheit der Dentalspule 26 können insbesondere von einer Halterung 35 und/oder einem Halteelement 33 getragen werden, welche an die äußere Gestalt der Kieferregion 43 des Patienten 15 angepasst sind (siehe Fig. 3).

Die dargestellte Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der Magnetfeld-erzeugenden Komponenten aufweist. Die Magnetresonanzvorrichtung 10 kann insbesondere eine dedizierte Magnetresonanzvorrichtung 10 sein, welche dazu ausgebildet ist, eine Magnetresonanzbildgebung der Kieferregion eines stehenden oder sitzenden Patienten 15 durchzuführen.

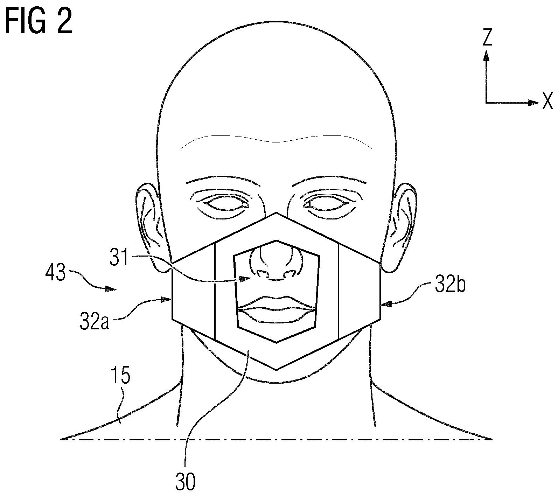

Fig. 2 zeigt eine Ausführungsform der erfindungsgemäßen Dentalspule 26, bei welcher das erste Element 30 sowie das zweite Element 32 in der anwendungsgemäßen Position an der Kieferregion 43 des Patienten 15 positioniert sind. Das erste Element 30 ist in dem vorliegenden Beispiel mechanisch mit dem zweiten Element 32 verbunden, wobei die beiden Flügel oder Flanken 32a und 32b des zweiten Elements 32 unter einem Winkel zu dem ersten Element 30 angeordnet sind. Der rechte Flügel 32a ist dabei einer Oberflächenkontur einer rechten Wangenregion des Patienten 15 nachgeformt, während der linke Flügel 32b der Oberflächenkontur einer linken Wangenregion des Patienten 15 nachgeformt ist. Ein Flügel 32a oder 32b des zweiten Elements 32 kann auch ein drittes Element darstellen. Das erste Element 30 und/oder das zweite Element 32 können in einem geringen Abstand zu der Hautoberfläche der Kieferregion 43 des Patienten 15 positioniert sein oder an dieser anliegen.

Wie in Fig. 2 gezeigt, weist das erste Element 30 zumindest eine Aussparung 31 auf, welche dazu ausgebildet ist, die Nase und den Mund des Patienten 15 aufzunehmen. Das erste Element 30 besteht aus einem formsteifen bzw. rigiden Material, welches vorzugsweise der Mund- und/oder Nasenregion des Patienten 15 nachgeformt ist. Das flexible Element ist in dem dargestellten Beispiel aus einem elastischen Material gefertigt, in welches eine Antenne des zweiten Elements 32 eingebettet ist. Vorzugsweise weist die Antenne flexible oder biegbare Signalleiter auf, damit das zweite Element 32 der Kieferregion des Patienten 15 nachgeformt werden kann.

In Fig. 3 ist eine Ausführungsform der erfindungsgemäßen Dentalspule 26 dargestellt, bei welcher ein oberer Teil der Halterung 35a der Dentalspule 26 gegenüber einem unteren Teil der Halterung 35b und/oder einer Positionierungseinheit 44 der Dentalspule 26 schwenkbar gelagert ist. Die Dentalspule 26 weist in dem gezeigten Beispiel einen Schwenkmechanismus 38 auf, welcher vorliegend ein Drehlager aufweist. Der obere Teil der Halterung 35a mit dem ersten Element 30 kann entsprechend um einen Angelpunkt, welcher durch eine Position des Drehlagers definiert ist, gegenüber der Patientenlagerungsvorrichtung 16 und/oder dem Patienten 15 geschwenkt oder verkippt werden. Die Dentalspule 26 weist in der gezeigten Ausführungsform ferner ein Dämpfungselement 37 auf, welches ein unkontrolliertes Zuklappen des oberen Teils der Halterung 35a in Richtung des Patienten 15 begrenzt oder verhindert. Das Dämpfungselement 37 setzt den Rastelementen 36i, welche beispielsweise als Nocken, Nippel oder Auswölbungen ausgeführt sein können, bei dem Schwenken des oberen Teils der Halterung 35a einen vorbestimmten Widerstand entgegen. Ferner weist der obere Teil der Halterung 35a ein Rastelement 36z auf, welches eine Bewegung des oberen Teils der Halterung 35a in Richtung des Patienten 15 terminiert und den oberen Teil der Halterung 35a in einer vorbestimmten Relativposition zu dem Patienten 15 feststellt.

Die Positionierungseinheit 44 ist dazu ausgelegt, die Dentalspule 26 relativ zu der Patientenlagerungsfläche der Patientenlagerungsvorrichtung 16 und/oder dem Patienten 15 zu positionieren. Die Positionierungseinheit 44 kann wie gezeigt entlang eines geeigneten Führungselements 45 positioniert werden. Die Positionierungseinheit 44 ist mechanisch mit einem unteren Teil der Halterung 35b verbunden, welche mittels des Schwenkmechanismus 38 mit dem oberen Teil der Halterung 35a verbunden ist. Der obere Teil der Halterung 35a kann ebenso als ein Halteelement 33 für das erste Element 30 und/oder das zweite Element 32 betrachtet werden.

Die in Fig. 3 gezeigte Ausführungsform der erfindungsgemäßen Dentalspule 26 weist ferner einen Führungsmechanismus 41 für eine Feinjustierung einer Position des ersten Elements 30 auf. Eine Führungsachse der Schraube 41a ist bei anwendungsgemäßer Positionierung der Dentalspule 26 an der Kieferregion 43 des Patienten 15, also beispielsweise in einem zugeklappten Zustand des oberen Teils der Halterung 35a, im Wesentlichen parallel zu einer Schnittlinie einer Sagittalebene und einer Transversalebene an der Kieferregion 43 des Patienten 15 ausgerichtet.

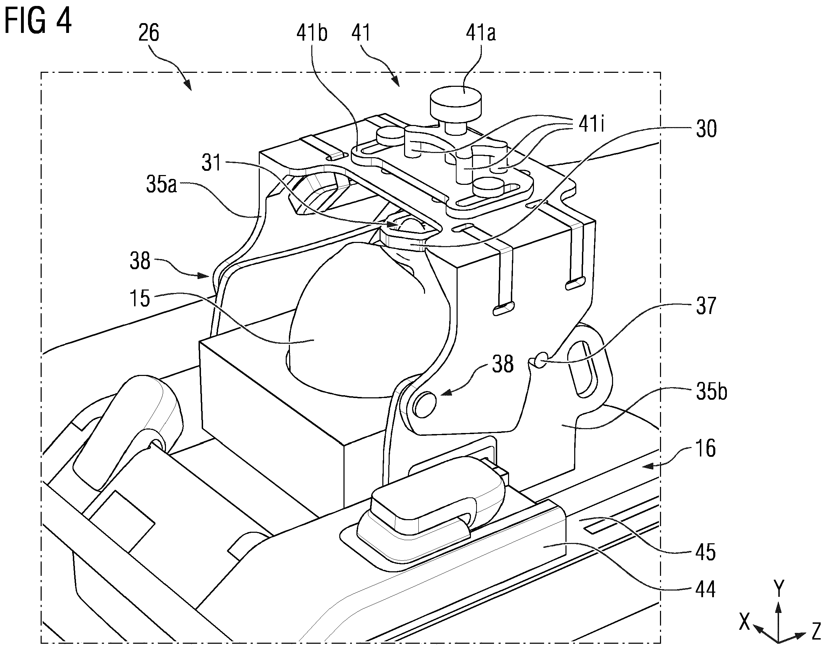

In Fig. 4 ist eine alternative Ansicht der in Fig. 3 gezeigten Ausführungsform der erfindungsgemäßen Dentalspule 26 dargestellt. Der Führungsmechanismus 41 kann beispielsweise Stifte oder Bolzen 41i aufweisen, welche den oberen Teil der Halterung 35a durchbrechen und mit dem ersten Element 30 mechanisch verbunden sind. Der Führungsmechanismus 41 ist dazu ausgebildet, das erste Element 30 entlang der Y-Richtung zu positionieren, wenn die Schraube 41a gedreht wird. Die Bolzen oder Stifte 41i des Führungsmechanismus 41 sind hierbei durch Langlöcher in dem oberen Teil der Halterung 35a geführt und lassen sich mittels der Schraubplatte 41b zumindest entlang der Z-Richtung relativ zu dem Patienten 15 positionieren. Der Führungsmechanismus 41 kann ferner auch als ein Halteelement 33 interpretiert werden, welches dazu ausgebildet ist, das erste Element 30 in der anwendungsgemäßen Position an der Kieferregion 43 des Patienten 15 zu halten und/oder das erste Element 30 relativ zu der Kieferregion 43 des Patienten 15 zu positionieren.

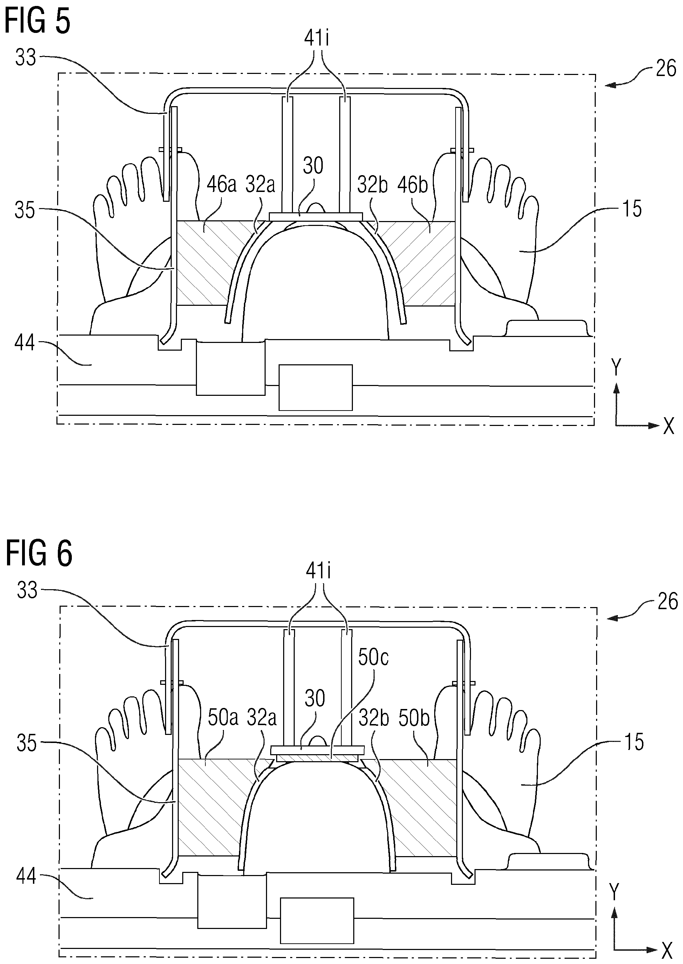

Fig. 5 zeigt eine Ausführungsform der erfindungsgemäßen Dentalspule 26, bei welcher das zweite Element 32 mittels der flexiblen Halteelemente 46a und 46b einer äußeren Gestalt der Kieferregion 43 des Patienten 15 nachgeformt ist. Die flexiblen Halteelemente 46a und 46b können beispielsweise aus einem elastischen Schaumstoff bestehen, welcher mit einer erwarteten Geometrie der Kieferregion 43 einer Patientengruppe abgestimmt ist. Vorzugsweise werden die flexiblen Halteelemente 46a und 46b zwischen der Halterung 35 und den beiden Flügeln 32a und 32b des zweiten Elements 32 eingespannt oder eingeklemmt, sodass die beiden Flügel 32a und 32b in einer anwendungsgemäßen Position in einem vorbestimmten Abstand zu der Hautoberfläche der Kieferregion 43 des Patienten 15 gehalten werden. Das flexible Element des zweiten Elements 32 wird bei der anwendungsgemäßen Positionierung eines flexiblen Halteelements 46 vorzugsweise derart verformt, dass eine Kontur einer oder mehrerer Antennen des zweiten Elements 32 einer Kontur der Kieferregion 43 des Patienten 15 nachgeformt werden. Das zweite Element 32 wird dabei mittels des flexiblen Halteelements 46 relativ zu dem ersten Element 30 verformt und/oder positioniert.

Das erste Element 30 ist dabei wie in Fig. 4 gezeigt mittels des Anpassungsmechanismus 41 auf der Hautoberfläche oder in einem geringen Abstand zu der Hautoberfläche der Kieferregion 43 des Patienten 15 positionierbar. Die flexiblen Halteelemente 46a und 46b können auch als Halteelement 33 verstanden werden, welches das zweite Element 32 der Kieferregion des Patienten nachformt und in einer anwendungsgemäßen Position an der Kieferregion hält.

Fig. 6 zeigt eine alternative Ausführungsform der erfindungsgemäßen Dentalspule, bei welcher das zweite Element 32 getrennt von dem ersten Element 30 vorliegt. Die Flügel 32a und 32b des zweiten Elements 32 können dabei mechanisch mit einer Basis der Halterung 35 (z. B. einer ersten Fläche) oder der Positionierungseinheit 44 verbunden sein. Hierbei können die flexiblen Halteelemente 46a, 46b der Fig. 5 auch als Fixierungselemente 50a, 50b und 50c fungieren, welche den Kopf des Patienten 15 durch ein Andrücken der beiden Flügel 32a und 32b des zweiten Elements 32 auf die Hautoberfläche des Patienten 15 in einer vorbestimmten Relativposition zu dem ersten Element 30 fixieren.

In dem vorliegenden Beispiel weist das erste Element 30 auf einer der Kieferregion 43 des Patienten 15 zugewandten Seite ein Fixierungselement 50c auf, welches aus einem elastischen Material besteht. Das erste Element 30 wird in dieser Ausführungsform vorzugsweise mittels des Führungsmechanismus 41 mit der Hautoberfläche der Kieferregion 43 des Patienten 15 in Kontakt gebracht, sodass der Kopf des Patienten 15 durch das erste Element 30 in einer vorbestimmten Relativposition zu der Dentalspule 26 fixiert ist. Das elastische Material des Fixierungselements 50c kann dazu ausgebildet sein, den Druck des Führungsmechanismus 41 auf die Kieferregion 43 des Patienten 15 zu verteilen und einen Komfort des Patienten 15 während einer Magnetresonanzmessung zu erhöhen.

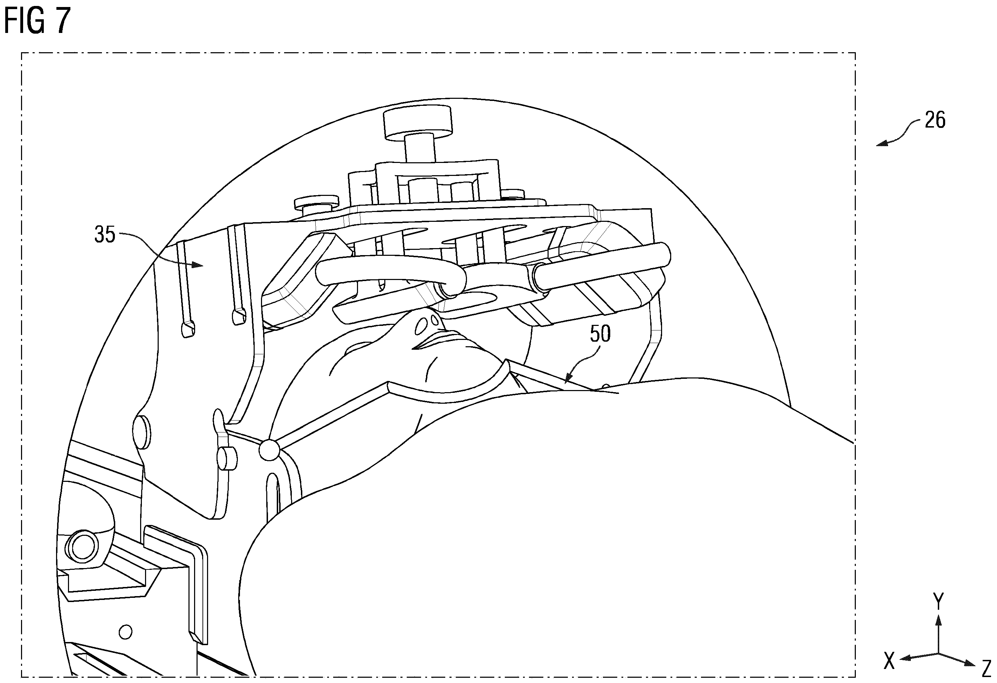

Fig. 7 zeigt eine alternative Ausführungsform einer erfindungsgemäßen Dentalspule 26 mit einem Fixierungselement 50. In dem vorliegenden Beispiel umfasst das Fixierungselement 50 ein elastisches Band, welches das Kinn bzw. den Unterkiefer des Patienten 15 umgreift. Das elastische Band ist vorzugsweise mechanisch mit der Halterung 35 verbunden. Durch das elastische Auslenken des elastischen Bands bei der Positionierung des Fixierungselements 50 an dem Kinn bzw. des Unterkiefers des Patienten 15 treten elastische Rückstellkräfte in dem elastischen Band auf, welche den Kopf des Patienten stabilisieren oder in einer vorbestimmten Relativposition zu der Dentalspule 26 fixieren. Es ist weiterhin vorstellbar, dass das elastische Band des Fixierungselements 50 mit einem Zugmechanismus (nicht gezeigt) verbunden ist, welcher dazu ausgebildet ist, das elastische Band mit einer größeren Kraft gegen den Kopf des Patienten 15 anzudrücken.

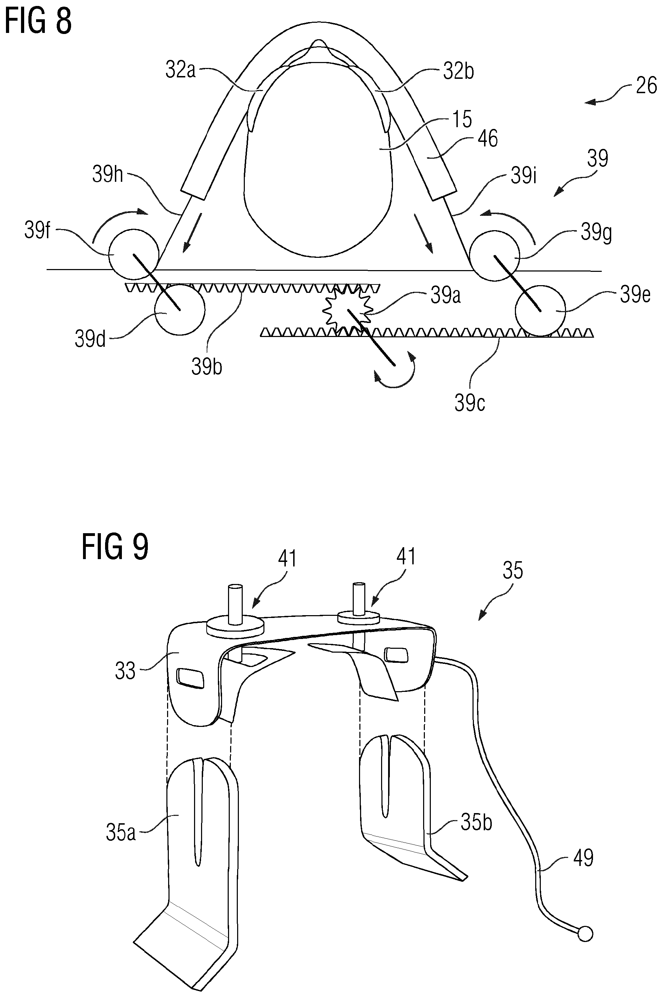

Fig. 8 zeigt eine Ausführungsform der erfindungsgemäßen Dentalspule 26, bei welcher zumindest das zweite Element 32 mechanisch mit einem flexiblen Halteelement 46 verbunden ist. Das flexible Halteelement 46 ist dazu ausgebildet, das zweite Element 32 an die Kieferregion des Patienten 15 anzuformen und in einer anwendungsgemäßen Position für die Durchführung einer Magnetresonanzmessung der Kieferregion 43 zu halten. Das flexible Halteelement 46 weist vorliegend ein elastisches Material mit einer im Wesentlichen quaderförmigen Gestalt auf, welche die Kieferregion 43 des Patienten 15 umschließt und die beiden Flügel 32a und 32b des zweiten Elements 32 an die Kieferregion 43 anformt. Die Dentalspule 26 weist einen Zugmechanismus 39 auf, welcher mechanisch mit einem ersten Ende des flexiblen Halteelements 46 an der linken Wangenregion und einem zweiten Ende des flexiblen Halteelements 46 an der rechten Wangenregion des Patienten 15 verbunden ist. Die Zugkraft wird durch Betätigung, insbesondere Drehung, eines zentralen Getriebeelements 39a bereitgestellt. Das zentrale Getriebeelement 39a ist dazu ausgebildet, zwei Getriebestangen 39b und 39c zu drehen oder relativ zueinander zu positionieren, sodass zwei Zuganker 39d und 39e gedreht oder relativ zueinander bewegt werden. Die beiden Zuganker 39d und 39e sind ferner jeweils mit einer Federrolle 39f und 39g verbunden, welche die Zugelemente 39h und 39i unter Spannung halten und/oder eine Zugkraft bzw. eine Richtung der Zugkraft von den Zugankern 39d und 39e auf das Halteelement 33 übertragen. Die Zugkraft, welche mittels des Zugmechanismus 39 bereitgestellt wird, ist derart orientiert, dass die Flügel 32a und 32b des zweiten Elements 32 mittels des flexiblen Haltelements 46 an die Kieferregion des Patienten 15 angedrückt werden. Das erste Element 30 kann entsprechend dem zweiten Element 32 an dem flexiblen Halteelement 46 positioniert sein oder separat davon an der Kieferregion 43 des Patienten 15 positioniert werden. Es ist insbesondere vorstellbar, dass das flexible Halteelement 46 eine Aussparung oder einen Durchbruch aufweist, welcher dazu ausgebildet ist, das erste Element 30 aufzunehmen, sodass das erste Element 30 unabhängig von dem flexiblen Halteelement 46 an der Kieferregion 43 des Patienten positionierbar ist.

Fig. 9 zeigt eine Ausführungsform einer Halterung 35 der erfindungsgemäßen Dentalspule 26. Die Halterung 35 weist vorliegend zwei Trägerelemente 35a und 35b auf, welche den Kopf des Patienten 15 (nicht gezeigt) bei anwendungsgemäßer Positionierung der Dentalspule 26 flankieren. Die Halterung 35 weist ferner ein Halteelement 33 auf, welches dazu ausgebildet ist, das erste Element 30 (nicht gezeigt) und/oder das zweite Element 32 (nicht gezeigt) an der Kieferregion 43 des Patienten 15 zu halten. Das Halteelement 33 weist insbesondere einen Anpassungsmechanismus 41 auf, welcher dazu ausgebildet ist, zumindest das zweite Element 32 (oder ein drittes Element) der Kieferregion 43 des Patienten 15 nachzuformen. Das Halteelement 33 ist in dem gezeigten Beispiel besonders einfach von der Halterung 35 zu lösen, um einen Zugang des Kopfs des Patienten 15 zu einem Volumen zwischen den Trägerelementen 35a und 35b zu ermöglichen.

Das Halteelement 33 ist im vorliegenden Beispiel mit einem Sicherheitsmechanismus 49 verbunden, welcher als eine Reißleine ausgebildet ist. Die Reißleine kann dazu ausgebildet sein, eine mechanische Verbindung zwischen der Halterung 35 und dem Halteelement 33 zu lösen, wenn eine vorbestimmte Zugkraft an der Reißleine überschritten wird. Das Halteelement 33 lässt sich somit in einem Notfall mittels der Reißleine von der Halterung 35 lösen. Der Sicherheitsmechanismus 49 kann alternativ auch als ein Knopf, ein Schalter oder dergleichen ausgeführt sein. Der Sicherheitsmechanismus 49 kann insbesondere dazu ausgebildet sein, ein Stellelement anzusteuern oder zu betätigen, welches eine mechanische Verbindung zwischen dem Halteelement 33 und der Halterung 35 löst. Der Sicherheitsmechanismus 49 kann hierfür auch eine Kamera und/oder ein Mikrofon aufweisen, welche dazu ausgebildet sind, eine Geste und/oder eine Sprachnachricht einer Person zu erfassen. Die Geste und/oder die Sprachnachricht der Person können dabei einen Wunsch zur Überführung der Dentalspule 26 in eine Öffnungsposition kodieren.

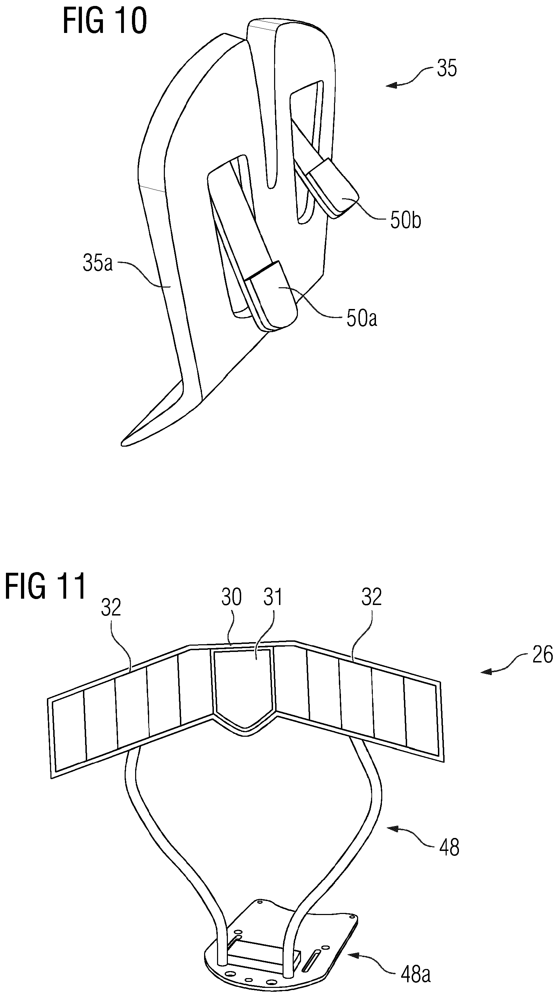

Fig. 10 zeigt eine Ausführungsform eines Trägerelements 35a der erfindungsgemäßen Dentalspule 26. Das Trägerelement 35a weist in dem gezeigten Beispiel zwei Fixierungselemente 50a und 50b auf, welche schwenkbar an dem Trägerelement 35a gelagert sind und in einer vorbestimmten Relativposition zu dem Trägerelement 35a fixiert oder festgestellt werden können. Die Fixierungselemente 50a und 50b weisen an in Richtung des Patienten 15 weisenden Enden elastische Kissen auf, welche bei anwendungsgemäßer Positionierung der Dentalspule 26 seitlich an dem Kopf des Patienten 15 und/oder dem Hinterkopf des Patienten 15 positioniert sind. Die Fixierungselemente 50a und 50b sind insbesondere dazu ausgebildet, den Kopf des Patienten während einer Magnetresonanzmessung zu stützen bzw. eine Bewegung des Kopfs zu begrenzen oder zu verhindern.

In Fig. 11 ist eine Ausführungsform der erfindungsgemäßen Dentalspule 26 dargestellt, bei welcher das erste Element 30 und das zweite Element 32 mechanisch mit einer Schwanenhalsmechanik 48 verbunden sind. Die Schwanenhalsmechanik 48 kann eine Grundplatte 48a aufweisen, welche mit einer Halterung 35 oder einer Positionierungseinheit 44 verbunden ist. Das erste Elemente 30 ist vorliegend mechanisch mit dem zweiten Element 32 verbunden. Das zweite Element 32 ist aufgrund des flexiblen Elements verformbar und lässt sich gegenüber dem ersten Element 30 abwinkeln oder verbiegen, um ein Nachformen der Kieferregion 43 des Patienten 15 zu ermöglichen.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Dentalspule (26), umfassend eine Halterung (35), ein erstes Element (30) und ein zweites Element (32), wobei das erste Element (30) aus einem formsteifen Material besteht und eine Aussparung (31) umfasst, welche dazu geeignet ist, eine Mundregion und/oder eine Nasenregion eines Patienten (15) bei anwendungsgemäßer Positionierung der Dentalspule (26) an einer Kieferregion (43) des Patienten (15) aufzunehmen, wobei das zweite Elemente (32) ein flexibles Element aufweist, welches dazu ausgebildet ist, ein Nachformen der Kieferregion (43) des Patienten (15) mittels des flexiblen Elements zu ermöglichen, wobei das erste Element (30) und das zweite Element (32) eine Antenne aufweisen, welche dazu ausgebildet ist, hochfrequente Signale in einem Frequenz- und Leistungsbereich einer Magnetresonanzmessung zu empfangen, wobei die Halterung (35) mechanisch mit dem ersten Element (30) und/oder dem zweiten Element (32) verbunden ist, wobei das erste Element (30) und/oder das zweite Element (32) mittels der Halterung (35) relativ zu dem Patienten (15) positionierbar sind und wobei die Halterung (35) dazu ausgebildet ist, das erste Element (30) und/oder das zweite Element (32) in der anwendungsgemäßen Position an der Kieferregion (43) des Patienten (15) zu halten, **dadurch gekennzeichnet, dass** die Halterung (35) einen Führungsmechanismus (41) mit einer Führungsachse aufweist, wobei die Führungsachse im Wesentlichen parallel zu einer Schnittlinie einer Sagittalebene und einer Transversalebene an der Kieferregion (43) des Patienten (15) ausgerichtet ist, wobei der Führungsmechanismus (41) dazu ausgebildet ist, das erste Element (30) relativ zu der Halterung (35) entlang der Führungsachse zu positionieren, um einen Abstand zwischen dem ersten Element (30) und der Kieferregion (43) des Patienten (15) einzustellen.

2. Dentalspule (26) nach Anspruch 1, wobei das zweite Element (32) unabhängig von dem ersten Element (30) positionierbar ist oder wobei das erste Element (30) und das zweite Element (32) mechanisch miteinander verbunden sind und wobei das zweite Element (32) von dem ersten Element (30) gehalten wird.

3. Dentalspule (26) nach einem der vorhergehenden Ansprüche, ferner aufweisend einen Anpassungsmechanismus, welcher dazu ausgebildet ist, ein Anpassen einer Form des flexiblen Elements vorzunehmen, um das zweite Element (32) der Kieferregion (43) des Patienten (15) nachzuformen.

4. Dentalspule (26) nach Anspruch 3, wobei das flexible Element ein intelligentes Material aufweist, welches dazu ausgebildet ist, eine Materialeigenschaft in Abhängigkeit eines vorbestimmten Anregungssignals zu verändern, wobei der Anpassungsmechanismus dazu ausgebildet ist, das Anpassen der Form des zweiten Elements (32) mittels einer Übertragung des Anregungssignals auf das flexible Element zu unterstützen.

5. Dentalspule (26) nach einem der vorhergehenden Ansprüche, ferner aufweisend eine Positionierungseinheit (44), welche dazu ausgebildet ist, eine Position und/oder Orientierung der Halterung (35) relativ zu dem Patienten (15) und/oder einer Patientenlagerungsvorrichtung (16) einzustellen.

6. Dentalspule (26) nach einem der vorhergehenden Ansprüche, wobei die Halterung (35) einen Schwenkmechanismus (38) aufweist und wobei das erste Element (30) und/oder das zweite Element (32) bei anwendungsgemäßer Positionierung der Dentalspule (26) an der Kieferregion (43) des Patienten (15) mittels des Schwenkmechanismus (38) um einen maximalen Winkel schwenkbar gegenüber dem Patienten (15) gelagert sind, wobei der maximale Winkel des ersten Elements (30) und/oder des zweiten Elements (32) gegenüber dem Patienten (15) so gewählt ist, dass ein Kopf des Patienten (15) ungehindert in einer anwendungsgemäßen Relativposition zu der Halterung (35) positioniert werden kann.

7. Dentalspule (26) nach einem der vorhergehenden Ansprüche, wobei die Halterung (35) eine Schwanenhalsmechanik (48) oder einen Gelenkschlauch aufweist.

8. Dentalspule (26) nach Anspruch 6, wobei der Schwenkmechanismus (38) ferner ein Dämpfungselement (37) aufweist, welches dazu ausgebildet ist, eine Bewegung des ersten Elements (30) und/oder des zweiten Elements (32) bei Schwenkung des ersten Elements und/oder des zweiten Elements (32) entlang einer vorbestimmten Wegstrecke abzudämpfen und/oder zu begrenzen.

9. Dentalspule (26) nach Anspruch 3, ferner umfassend ein flexibles Halteelement (46), welches bei anwendungsgemäßer Positionierung der Dentalspule (26) an der Kieferregion (43) des Patienten (15) zumindest mit dem zweiten Element (32) verbunden ist, wobei der Anpassungsmechanismus als ein Zugmechanismus (39) ausgestaltet ist, welcher mechanisch mit dem flexiblen Halteelement (46) verbunden ist und dazu ausgelegt ist, eine Zugkraft bereitzustellen, welche das flexible Halteelement (46) in eine Richtung der Kieferregion (43) des Patienten (15) führt.

10. Dentalspule (26) nach einem der vorhergehenden Ansprüche, ferner aufweisend ein drittes Element mit einem flexiblen Element, wobei das flexible Element dazu ausgebildet ist, ein Nachformen des dritten Elements der Kieferregion (43) des Patienten (15) zu ermöglichen und wobei das dritte Element bei anwendungsgemäßer Positionierung der Dentalspule (26) auf einer dem zweiten Element (32) gegenüberliegenden Seite der Kieferregion (43) des Patienten (15) angeordnet ist, wobei das dritte Element eine Antenne aufweist, welche dazu ausgebildet ist, hochfrequente Signale in einem Frequenz- und Leistungsbereich einer Magnetresonanzmessung zu empfangen.

11. Dentalspule (26) nach einem der vorhergehenden Ansprüche, ferner aufweisend ein Fixierungselement (50), welches dazu ausgebildet ist, die Halterung (35) und/oder das erste Element (30) mechanisch mit einem Kopf des Patienten (15) zu verbinden, um den Kopf des Patienten (15) in einer vorbestimmten Relativposition zu dem ersten Element (30) zu fixieren.

12. Dentalspule (26) nach einem der vorhergehenden Ansprüche, ferner aufweisend einen Sicherheitsmechanismus (49), welcher dazu ausgelegt ist, die Dentalspule (26) bei einer Betätigung des Sicherheitsmechanismus (49) durch eine Person in eine Öffnungsposition zu überführen, um die Dentalspule (26) aus der anwendungsgemäßen Position an der Kieferregion (43) des Patienten (15) zu entfernen.

13. Magnetresonanzvorrichtung (10), umfassend eine Dentalspule (26) nach einem der vorhergehenden Ansprüche, wobei die Magnetresonanzvorrichtung (10) dazu ausgebildet ist, hochfrequente Signale in einem Frequenz- und Leistungsbereich einer Magnetresonanzmessung aus einer Kieferregion (43) eines Patienten (15) mittels der Dentalspule (26) zu erfassen, um eine Bildgebung der Kieferregion (43) des Patienten (15) durchzuführen.

## Claims

1. Dental coil (26) comprising a retainer (35), a first element (30) and a second element (32), wherein the first element (30) consists of a dimensionally stable material and comprises a recess (31) which is suitable for receiving a mouth region and/or a nose region of a patient (15) when the dental coil (26) is positioned in a manner appropriate for use on a jaw region (43) of the patient (15), wherein the second element (32) has a flexible element which is designed to allow it to be shaped to conform to the contour of the jaw region (43) of the patient (15) by means of the flexible element, wherein the first element (30) and the second element (32) have an antenna which is embodied to receive radiofrequency signals in a frequency and power range of a magnetic resonance measurement, wherein the retainer (35) is mechanically connected to the first element (30) and/or the second element (32), wherein the first element (30) and/or the second element (32) can be positioned relative to the patient (15) by means of the retainer (35) and wherein the retainer (35) is embodied to hold the first element (30) and/or the second element (32) in the position appropriate for use on the jaw region (43) of the patient (15), **characterised in that** the retainer (35) has a guide mechanism (41) having a guide axis, wherein the guide axis is aligned substantially parallel to a line of intersection of a sagittal plane and a transverse plane on the jaw region (43) of the patient (15), wherein the guide mechanism (41) is embodied to position the first element (30) relative to the retainer (35) along the guide axis in order to set a distance between the first element (30) and the jaw region (43) of the patient (15).

2. Dental coil (26) according to claim 1, wherein the second element (32) can be positioned independently of the first element (30) or wherein the first element (30) and the second element (32) are mechanically connected to each other and wherein the second element (32) is held in place by the first element (30).

3. Dental coil (26) according to one of the preceding claims, further having an adjustment mechanism which is embodied to adjust a shape of the flexible element in order to shape the second element (32) to conform to the contour of the jaw region (43) of the patient (15).

4. Dental coil (26) according to claim 3, wherein the flexible element has a smart material which is embodied to modify a material property as a function of a predetermined excitation signal, wherein the adjustment mechanism is embodied to assist the matching of the shape of the second element (32) by means of a transmission of the excitation signal to the flexible element.

5. Dental coil (26) according to one of the preceding claims, further having a positioning unit (44) which is embodied to set a position and/or an orientation of the retainer (35) relative to the patient (15) and/or to a patient support and positioning device (16).

6. Dental coil (26) according to one of the preceding claims, wherein the retainer (35) has a pivoting mechanism (38) and wherein the first element (30) and/or the second element (32) are mounted so as to be pivotable by a maximum angle relative to the patient (15) by means of the pivoting mechanism (38) when the dental coil (26) is positioned in a manner appropriate for use on the jaw region (43) of the patient (15), wherein the maximum angle of the first element (30) and/or the second element (32) relative to the patient (15) is chosen such that a head of the patient (15) can be positioned unobstructed in a position appropriate for use relative to the retainer (35).

7. Dental coil (26) according to one of the preceding claims, wherein the retainer (35) has a swan-neck mechanism (48) or a joint hose.

8. Dental coil (26) according to claim 6, wherein the pivoting mechanism (38) further has a damping element (37) which is embodied to dampen and/or limit a movement of the first element (30) and/or the second element (32) when the first element and/or the second element (32) are/is pivoted along a predetermined path of travel.

9. Dental coil (26) according to claim 3, further comprising a flexible holding element (46) which is connected at least to the second element (32) when the dental coil (26) is positioned in a manner appropriate for use on the jaw region (43) of the patient (15), wherein the adjustment mechanism is designed as a pull mechanism (39) which is mechanically connected to the flexible holding element (46) and is configured to provide a tractive force which guides the flexible holding element (46) in a direction of the jaw region (43) of the patient (15).

10. Dental coil (26) according to one of the preceding claims, further having a third element with a flexible element, wherein the flexible element is embodied to allow the third element to be shaped to conform to the contour of the jaw region (43) of the patient (15) and wherein the third element is arranged on a side of the jaw region (43) of the patient (15) lying opposite the second element (32) when the dental coil (26) is positioned in a manner appropriate for use, wherein the third element has an antenna which is embodied to receive radiofrequency signals in a frequency and power range of a magnetic resonance measurement.

11. Dental coil (26) according to one of the preceding claims, further having a fixing element (50) which is embodied to mechanically connect the retainer (35) and/or the first element (30) to the head of the patient (15) in order to fix the head of the patient (15) in place in a predetermined position relative to the first element (30).

12. Dental coil (26) according to one of the preceding claims, further having a safety mechanism (49) which is configured to transfer the dental coil (26) into an open position when the safety mechanism (49) is actuated by a person in order to remove the dental coil (26) from the position appropriate for use on the jaw region (43) of the patient (15).

13. Magnetic resonance device (10) comprising a dental coil (26) according to one of the preceding claims, wherein the magnetic resonance device (10) is embodied to record radiofrequency signals in a frequency and power range of a magnetic resonance measurement from a jaw region (43) of a patient (15) by means of the dental coil (26) in order to perform an imaging scan of the jaw region (43) of the patient (15).

## Revendications

1. Bobine (26) dentaire, comprenant une fixation (35), un premier élément (30) et un deuxième élément (32), dans laquelle le premier élément (30) est en un matériau de forme rigide et comprend un évidement (31), qui est propre à recevoir une région buccale et/ou une région nasale d'un patient (15) lorsque la bobine (26) dentaire est en position conformément à l'utilisation sur une région (43) de la mâchoire du patient (15), dans laquelle le deuxième élément (32) a un élément souple, qui est constitué pour rendre possible une reproduction de la région (43) de la mâchoire du patient (15) au moyen de l'élément souple, dans laquelle le premier élément (30) et le deuxième élément (32) ont une antenne, qui est constituée pour recevoir des signaux de haute fréquence dans un domaine de fréquence et de puissance d'une mesure de résonnance magnétique, dans laquelle la fixation (35) est assemblée mécaniquement au premier élément (30) et/ou au deuxième élément (32), dans laquelle le premier élément (30) et/ou le deuxième élément (32) peuvent être mis en position par rapport au patient (15) au moyen de la fixation (35) et dans laquelle la fixation (35) est constituée pour maintenir le premier élément (30) et/ou le deuxième élément (32) en la position conforme à l'utilisation sur la région (43) de la mâchoire du patient (15), **caractérisée en ce que** la fixation (35) a un mécanisme (41) de guidage ayant un axe de guidage, dans laquelle l'axe de guidage est dirigé sensiblement parallèlement à une ligne d'intersection d'un plan sagittal et d'un plan transversal à la région (43) de la mâchoire du patient (15),
dans laquelle le mécanisme (41) de guidage est constitué pour mettre le premier élément (30) en position par rapport à la fixation (35) le long de l'axe de guidage, afin de régler une distance entre le premier élément (30) et la région (43) de la mâchoire du patient (15).

2. Bobine (26) dentaire suivant la revendication 1, dans laquelle le deuxième élément (32) peut être mis en position indépendamment du premier élément (30) ou dans laquelle le premier (30) et le deuxième élément (32) sont assemblés mécaniquement l'un à l'autre et dans laquelle le deuxième élément (32) est maintenu par le premier élément (30).

3. Bobine (26) dentaire suivant l'une des revendications précédentes, comportant en outre un mécanisme d'adaptation, qui est constitué pour effectuer une adaptation d'une forme de l'élément souple, afin que le deuxième élément (32) reproduise la région (43) de la mâchoire du patient (15).

4. Bobine (26) dentaire suivant la revendication 3, dans laquelle l'élément souple comprend un matériau intelligent, qui est constitué pour modifier une propriété de matériau en fonction d'un signal d'excitation défini à l'avance, dans laquelle le mécanisme d'adaptation est constitué pour venir à l'appui de l'adaptation de la forme du deuxième élément (32) au moyen de la transmission du signal d'excitation à l'élément souple.

5. Bobine (26) dentaire suivant l'une des revendications précédentes, comprenant en outre une unité (44) de mise en position, qui est constituée pour régler une position et/ou une orientation de la fixation (35) par rapport au patient (15) et/ou à un dispositif (16) de couchette du patient.

6. Bobine (26) dentaire suivant l'une des revendications précédentes, dans laquelle la fixation (35) a un mécanisme (38) de pivotement et dans laquelle le premier élément (30) et/ou le deuxième élément (32) sont, lorsque la bobine (26) dentaire est mise en position conformément à l'utilisation sur la région (43) de la mâchoire du patient (15), montées au moyen du mécanisme (38) de pivotement pivotant d'un angle maximum par rapport au patient (15), dans laquelle l'angle maximum du premier élément (30) et/ou du deuxième élément (32) par rapport au patient (15) est choisi de manière à pouvoir mettre en position la tête du patient (15) sans obstacle dans une position relative conforme à l'utilisation par rapport à la fixation (35).

7. Bobine (26) dentaire suivant l'une des revendications précédentes, dans laquelle la fixation (35) a une mécanique (48) à col de cygne ou un tube souple articulé.

8. Bobine (26) dentaire suivant la revendication 6, dans laquelle le mécanisme (38) de pivotement a en outre un élément (37) d'amortissement, qui est constitué pour amortir et/ou limiter un déplacement du premier élément (30) et/ou du deuxième élément (32) lors du pivotement du premier élément et/ou du deuxième élément (32) le long d'une voie de déplacement définie à l'avance.

9. Bobine (26) dentaire suivant la revendication 3, comprenant en outre un élément (46) souple de maintien qui, lorsque la bobine (26) dentaire est en position conformément à l'utilisation sur la région (43) de la mâchoire du patient (15), est assemblé au moins au deuxième élément (32), dans laquelle le mécanisme d'adaptation est conformé sous la forme d'un mécanisme (39) de traction, qui est assemblé mécaniquement à l'élément (46) souple de maintien et est conçu pour donner une force de traction, qui guide l'élément (46) souple de maintien dans la direction de la région (43) de la mâchoire du patient (15).

10. Bobine (26) dentaire suivant l'une des revendications précédentes, comportant en outre un troisième élément ayant un élément souple, dans laquelle l'élément souple est constitué pour permettre une reproduction du troisième élément à la région (43) de la mâchoire du patient (15) et dans laquelle le troisième élément est, lorsque la bobine (26) dentaire est en position, conformément à l'utilisation, monté sur un côté, opposé au deuxième élément (32), de la région (43) de la mâchoire du patient (15), dans laquelle le troisième élément a une antenne, qui est conformée pour recevoir des signaux de haute fréquence dans une plage de fréquence et de puissance d'une mesure de résonnance magnétique.

11. Bobine (26) dentaire suivant l'une des revendications précédentes, comportant en outre un élément (50) d'immobilisation, qui est constitué pour assembler la fixation (35) et/ou le premier élément (30) mécaniquement à la tête du patient (15), afin d'immobiliser la tête du patient (15) dans une position relative définie à l'avance par rapport au premier élément (30).

12. Bobine (26) dentaire suivant l'une des revendications précédentes, comportant en outre un mécanisme (49) de sécurité, qui est conçu pour faire passer la bobine (26) dentaire, lorsque le mécanisme (49) de sécurité est actionné par une personne, dans une position d'ouverture, afin d'éloigner la bobine (26) dentaire de la position conforme à l'utilisation sur la région (43) de la mâchoire du patient (15).

13. Installation (10) de résonnance magnétique, comprenant une bobine (26) dentaire suivant l'une des revendications précédentes, dans laquelle l'installation (10) de résonnance magnétique est constituée pour détecter des signaux de haute fréquence dans une plage de fréquence et de puissance d'une mesure de résonnance magnétique d'une région (43) de la mâchoire d'un patient (15) au moyen de la bobine (26) dentaire, afin d'effectuer une imagerie de la région (43) de la mâchoire du patient (15).
